(19) Europäisches Patentamt
European Patent Office
Office européen des brevets

(11) **EP 3 640 948 A1**

(12) **EUROPÄISCHE PATENTANMELDUNG**

(43) Veröffentlichungstag:
**22.04.2020  Patentblatt 2020/17**

(51) Int Cl.:
***G16C 20/10*** *(2019.01)*     *G16C 10/00* *(2019.01)*

(21) Anmeldenummer: **18201344.1**

(22) Anmeldetag: **18.10.2018**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Covestro Deutschland AG
51373 Leverkusen (DE)**

(72) Erfinder: **Die Erfindernennung liegt noch nicht vor**

(74) Vertreter: **Levpat
c/o Covestro AG
Gebäude 4825
51365 Leverkusen (DE)**

(54) **MONTE-CARLO-METHODE ZUR AUTOMATISIERTEN UND HOCH-EFFIZIENTEN BERECHNUNG VON KINETISCHEN DATEN CHEMISCHER REAKTIONEN**

(57)   Die vorliegende Erfindung betrifft ein computenmplementiertes Verfahren zur Berechnung von Ubergangszustanden einer chemischen Reaktion, sowie ein System zur Datenveiarbeitung umfassend Mittel zur Aus- führung des Verfahrens, ein Computerprogramm umfassend Befehle die einen Computer veianlassen das Verfahren durchzuführen und die Verwendung des Computerprogramms

**EP 3 640 948 A1**

**Beschreibung**

Die vorliegende Erfindung betrifft ein computerimplementiertes Verfahren zur Berechnung von Ubergangszustanden einer chemischen Reaktion, sowie ein System zur Datenverarbeitung umfassend Mittel zur Ausführung des Verfahrens, ein Computerprogramm umfassend Befehle die einen Computer veranlassen das Verfahren durchzufuhren und die Verwendung des Computerprogramms

Wahrend einer chemischen Reaktion verandern die beteiligten Atome ihre Geometrie und Bindungen weiden gebrochen und neue Bindungen gebildet Dabei verandert sich auch die Energie dei beteiligten Atome und erreicht wahrend des Verlaufs der chemischen Reaktion einen Zustand maximaler Energie, den sogenannten Übergangszustand Der Ubergangszustand stellt einen Potentialwall oder eine Aktivierungsbarriere dar, der die Edukte von den Produkten der chemischen Reaktion tiennt. Ist die Aktivierungsbarriere uberwunden, so wird das Produkt gebildet Die Energie während einer chemischen Reaktion kann mit Hilfe einer sogenannten Potentialhyperflache dargestellt werden, welche die potentielle Energie der an der Reaktion beteiligten Atome in Abhangigkeit von ihrer Geometrie abbildet Dabei bildet die Potentialhyperflache auch Zustande ab, bei denen keine Produkte gebildet werden. Der direkte Reaktionspfad, bei dem Produkte unter Überwindung des Ubeigangszustandes gebildet werden, kann als Kurve gezeigt werden, bei der die Abstände dei einzelnen Atome der Moleküle gegen die Energie aufgetragen weiden Mit Hilfe von quantenchemischen Methoden und mathematischen Naherungsverfahren kann die Energie der Geometrie eines Moleküls oder eines Systems von mehreren Molekülen berechnet werden, wobei dei dabei zu betrachtende Funktionsraum die Freiheitsgrade des Molekuls umfasst Diese Verfahren ermöglichen die Ermittlung dei Geometrie am Ubergangszustand und erlauben Vorhersagen über die Reaktionskinetik einer chemischen Reaktion

Eine bekannte Methode mit der die Geometrie am Ubergangszustand ermittelt weiden kann, sind Newton-Raphson-Verfahren Die Berechnung von Ubergangszustanden mit Hilfe von Newton-Raphson-Verfahren weist jedoch einige Nachteile auf Zunachst kann ein Newton-Raphson Algorithmus nicht auf jede beliebige Ausgangsgeometrie für ein Molekul angewendet werden, sondern nui auf Molekulgeometrien, die bereits sehr nah an die Geometrie des Ubeigangszustandes angenähert sind Dies ist aufwendig, weil die Annaherung einer Molekülgeometrie an die Geometrie des Übergangszustandes ublicherweise handisch erfolgt, d.h es weiden Bmdungslangen pei Hand am Computer eingestellt Es sind auch Methoden bekannt, bei denen zunächst mit anderen Methoden Potentialhyperflachen berechnet werden aus denen mogliche Sattelpunkte identifiziert weiden Auch bei diesen Methoden muss also erst eine Geometrie ermittelt werden, die bereits sehr nah an die Geometrie des Ubeigangszustandes angenahert ist Aus diesen Grunden ist die Berechnung von Übergangszustanden mit Hilfe von Newton-Raphson-Algorithmen insbesondere für Molekule mit mehr als 100 Atomen mit der derzeitigen Computertechnik besonders zeit- und kostenintensiv Zui Anwendung von Newton Raphson Prozeduren und darauf aufbauenden Näherungen mussen außerdem zwingend zweite Ableitungen der Energie nach den Kernkoordinaten des betrachteten Molekuls berechnet werden Der Rechenaufwand der zweiten Ableitungen skaliert quadratisch mit der Große des Molekuls und ist deswegen für die Berechnung von Aktivierungsenergien geschwindigkeitsbestimmend.

Lin et al ("A flexible transition state searching method for atmospheric reaction systems," Chemical Physics 450-451, 2015, S 21-31) offenbaren eine Methode zur Untersuchung atmospharischer chemischer Reaktionen in der Gasphase Die Methode umfasst in einem ersten Schritt ein auf Monte-Carlo-Methoden basiertes Screening von Potentialhyperflachen mit Kraftfeld-Methoden, wobei genaherte Sattelpunkt-artige Regionen identifiziert werden Die verwendete Observable dei entsprechenden Monte Carlo Methode ist dabei dei Wert einer Energiefunktion Darauf aufbauend wird dann versucht mittels Newton-Raphson Methodenchemische Ubergangszustände zu lokalisieren Mit der hier offenbarten Monte-Carlo-Methode muss zunachst die gesamte Potentialhyperflache simuliert werden, bevor uberhaupt genaherte Sattelpunkt-artige Regionen identifiziert werden konnen Folglich konnen mit dieser Monte-Cailo-Methode nicht gezielt Sattelpunkt-artige Regionen optimiert werden Aus diesem Grund ist keine effiziente Nutzung der verwendeten Rechenressourcen zur Lokalisierung der benotigten Sattelpunkte moglich Dei Einsatz der hier offenbarten Methode beschrankt sich mit dei derzeitigen Computertechnik auf kleine Molekule mit 7 bis 30 Atomen Zudem werden mathematisch stark vereinfachte Methoden verwendet.

E Martinez-Núñez et al "An automated transition state search using classical trajectories initialized at multiple minima", Phys Chem Chem Phys, 14. Juni 2015, 17(22) 14912-21, "tsscds2018: A code foi automated discovery of chemical reaction mechanisms and solving the kinetics", J. Comp Chem, 24 September 2018, 39(23)1922-1930) offenbaren eine Methode zur Untersuchung chemische Reaktionspfade Dabei werden Potentialhyperflächen chemischer Molekule mit mathematisch vereinfachten Methoden berechnet. Anschließend wird versucht die chemisch relevanten Übergangszustande mit gewohnlichen Pseudo-Newton-Raphson-Methoden zu berechnen Auch hier ist nachteilig, dass zunächst die gesamte Potentialhyperflache berechnet werden muss, was zeitaufwandig ist und Rechnerleistung braucht Zudem kann auch diese Methode nur auf kleinere Molekule angewendet werden

Jacobson et al ("Automated Transition State Search and Its Application to Diverse Types of Organic Reactions", J. Chem Theory Comput. 13, 11, 5780-5797) offenbaren eine Methode zur automatisierten Berechnung von chemischen Ubergangszuständen Dabei werden chemische Ubeigangszustande aus chemischen Gleichgewichtszustanden berechnet Dieses Voi gehen benötigt eine separate Berechnung von chemischen Gleichgewichtszustanden, bevor eine Interpolation dei erhaltenen Geometrien durchgefuhrt weiden kann, mit welchen der Ubergangszustand angenahert wird In einem weiteren Schritt wird dann basieiend auf dieser Annaherung versucht eine Ubergangszustandsgeometrie zu berechnen Das Erstellen der benotigten Gleichgewichtszustande erfordert zusätzlichen Arbeitsaufwand durch den Fachmann Die in der offenbarten Methode verwendete Interpolation lasst sich nicht mit Erfolg auf beliebige Molekulgeometrien anwenden.

Hu et al ("A gradient-directed Monte Carlo method for global optimization in a discrete space Application to protein sequence design and folding" J Chem Phys 2009 Oct 21; 131(15) 154117) offenbaren eine Methode zur Berechnung von Proteinstrukturen offenbart Bei dieser Methode weiden Faltungen von Proteinstrukturen mittels Monte-Carlo-Methoden berechnet Zur Beschleunigung dei Konvergenz der Monte-Carlo-Prozeduien weiden Gradienten berechnet, welche in die Richtung und Große von Auslenkungen der Atompositionen eingehen Die Publikation lasst keine mögliche Verwendung dieser Methode zur gezielten Berechnung von Sattelpunkten erkennen

Es besteht somit ein Bedarf für ein Verfahren für die Berechnung von Ubergangszuständen, bei dem eine Molekulgeometrie fur eine Ubergangszustands einer chemischen Reaktion mit einem einfachen Verfahren, insbesondere einem computerimplementierten Verfahren, genahert werden kann Insbesondere besteht ein Bedarf für ein Verfahren mit dem Ubergangszustande ermittelt werden konnen, ohne zuvor eine Potentialhyperflache berechnen zu mussen. Insbesondere soll möglichst auf die Anwendung von pseudo-Newton-Raphson-Verfahien veizichtet weiden

Aufgabe dei vorliegenden Erfindung wai ein computerimplementiertes Verfahren zur Verfügung zu stellen, bei dem eine Geometrie eines Ubergangszustandes mit einem leicht anwendbaren, insbesondere einem computerimplementierten, Verfahren unter zur Hilfenahme einer quantenchemischen Methode berechnet wird Dabei soll bevorzugt möglichst kein quantenchemisches Verfahren verwendet werden, bei dem die zweite Ableitung der Energie nach den Kernkoordinaten des betrachteten Molekuls berechnet weiden muss, um den Ubeigangszustand berechnen zu konnen Insbesondere soll eine Molekülgeometrie für einen Ubergangszustand einer chemischen Reaktion ermittelt weiden konnen, ohne zuvor die Potentialhyperflache der chemischen Reaktion zu berechnen Insbesondere soll ein computerimplementiertes Verfahren zur Berechnung des Ubergangszustands fur Molekule mit bevorzugt mehr als 100 Atomen zui Verfügung gestellt weiden

Diese Aufgabe wurde gelost durch ein computerimplementiertes Verfahren zur Berechnung von Übergangszustanden einer chemischen Reaktion umfassend die Schritte

## A Erzeugen einer Startgeometrie

A1 Bereitstellung der dreidimensionalen Darstellung mindestens eines Molekuls im energetischen Grundzustand,
A2 Auswahl von mindestens einer Bindung des mindestens einen Molekuls und Auswahl einer Lange dei Bindung, wobei die ausgewahlte Lange nicht dei Länge der Bindung im energetischen Grundzustand des Molekuls entspricht, so dass eine Startgeometrie für die chemische Reaktion erhalten wird,
A3 Dreidimensionale Darstellung der Startgeometrie in kartesischen und/odei internen Koordinaten,

## B Ermittlung einer optimierten Startgeometrie

B1 Festlegung eines Funktionsraumes welcher die mindestens eine Bindung aus Schritt A2 und die durch diese Bindung verbundenen Atome umfasst,
B2 Geometrieoptimierung des in Schritt B1 ausgewahlten Funktionsraums mittels einer quantenchemischen Methode und mit der Randbedingung, dass die in Schritt A2 ausgewählte Lange dei mindestens einen Bindung konstant gehalten wird, so dass eine optimierte Startgeometrie erhalten wird,
B3 Ermittlung der Gradient-Norm B3 fur die optimierte Startgeometrie, wobei die Gradient-Norm durch die erste Ableitung einer Funktion E = f(x) der quantenchemischen Methode und erhalten wird, mit E=Gesamtenergie der optimierten Startgeometrie und x=Kernkoordinaten des Molekuls in der optimierten Startgeometrie,
B4.1 wenn die Gradient-Norm B3 $\nabla$ $0 \leq \nabla \leq 0,03$ $E_h$ $a_0^{-1}$ betragt, dann Einordnung dei optimierten Startgeometrie als eine Vorstufe für den Übergangszustand dei chemischen Reaktion und Fortführung des Verfahrens mit Schritt D1, oder
B4 2 wenn die Gradient-Norm B3 $\nabla$ > 0,03 $E_h$ $a_0^{-1}$ betragt, dann Ermittlung der Vorstufe fui den Ubergangszustand der chemischen Reaktion ausgehend von dei optimierten Startgeometrie durch ein Verfahren umfassend die folgenden Schritte.

## C Ermittlung der Vorstufe für den Übergangszustand der chemischen Reaktion

C1 Variation dei optimierten Startgeometrie unter Anwendung eines Monte-Carlo-Algorithmus wobei

C1 1 wenigstens ein Atom aus dem in Schritt B1 ausgewählten Funktionsraum zufällig ausgewahlt wird,

C1.2 ein Vektor fur eine Auslenkung des in Schritt C1 gewahlten Atoms zufällig ausgewahlt wird, wobei der zufällig ausgewahlte Betrag des Vektors mit dei Gradient-Norm B3 gewichtet wird,

C1 3 das Schritt C1 1 ausgewahlte Atom anhand des Vektors aus Schritt C1 2 aus der Position des Atoms in dei optimierten Startgeometrie ausgelenkt wird, so dass eine Vorstufe für einen Ubergangszustand der chemischen Reaktion erhalten wird,

C2 Geometrieoptimierung dei Vorstufe für den Ubergangszustand mittels dei quantenchemischen Methode und mit dei Randbedingung, dass die mindestens eine Bindung aus Schritt A2 die Lange hat, die sie in der Schritt C3 ermittelt wurde,

C3 Ermittlung dei Gradient-Norm C3 fur die Vorstufe für den Ubergangszustand aus Schritt C4, wobei die Gradient-Norm durch die erste Ableitung der Funktion $E = f(x)$ der quantenchemischen Methode und erhalten wird, mit E=Gesamteneigie der Vorstufe für den Übergangszustand und x=Kernkoordinaten des Molekuls in der Vorstufe für den Ubergangszustand,

C4.1 wenn die Gradient-Norm C3 $\nabla$ $0 \leq \nabla \leq 0,03$ $E_h$ $a_0^{-1}$ betragt, dann Fortführung des Verfahrens mit Schritt D1, oder

C4 2 wenn die Gradient-Noim C3 $\nabla > 0,03$ $E_h$ $a_0^{-1}$ betragt, dann Wiederholung dei Schritte C1 bis C3 bis eine Gradient-Norm C3 $\nabla$ $0 \leq \nabla \leq 0,03$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Ubergangszustand variiert wird, wenn der Wert ihrer Gradient-Norm C3 niedriger ist als der Wert der Gradient-Norm B3 dei optimierten Startgeometne, oder

(b) fur den Fall dass die Schritte C1 bis C3 mehr als ein Mal durchgefuhrt wurden, in Schritt C1 die Geometrie der optimierten Startgeometrie oder derjenigen Vorstufe für den Ubergangszustand aus den vorrangegangen Wiederholungen variiert wird, welche im Vergleich zu allen bisher erhaltenen Gradient-Normen C3 und B3 den niedrigsten Wert fui die Gradient-Norm C3 oder B3 aufweist,

**D Ermittlung des Übergangszustandes**

D1 Variation dei Vorstufe aus Schritt C4 1 oder dei Vorstufe aus Schritt B4 1, indem folgende Schritte für ein Atom innerhalb des in B1 definierten Funktionsraums durchgeführt werden

D1 1 Auswahl eines Atoms aus dem in Schritt B1 ausgewählten Funktionsraum,

D1 2 Auswahl eines Vektors für eine Auslenkung des in Schritt D1 1 gewahlten Atoms

D1 3 Auslenkung des in Schritt D1 1 ausgewahlten Atoms anhand des Vektors aus Schritt D1 2 aus seiner Position in dei Vorstufe, wobei die Position einmal um einen positiven Wert des Betrags des Vektors und einmal um einen negativen Wert des Betrags des Vektors ausgelenkt wird, so dass bei der Durchführung dei Schritte D1 1 bis D 1 3 zwei ausgelenkte Vorstufen erhalten weiden,

D2 Geometrieoptimierung der zwei ausgelenkten Vorstufen aus Schritt D1 mittels der quantenchemischen Methode unter dei Randbedingung dass die in D1 erhaltenen Bindungsabstande konstant gehalten werden, so dass zwei optimierte Vorstufen (1) und (11) erhalten werden,

D3 Berechnung dei Energie der zwei optimierten Vorstufen (1) und (11) aus Schritt D2 mittels des quantenchemischen Verfahrens,

D4 Vergleich des Weites der Energie der zwei optimierten Vorstufen mit dem Wert der Energie C3 oder B3 der Vorstufe, die in Schritt D1 verwendet wurde,

D4 1 wenn der Wert der Energie der optimierten Vorstufe (1) und der Wert der Energie der optimierten Vorstufe (11) kleiner sind als dei Wert der Energie C3 oder B3 dei Vorstufe, die in Schritt D1 verwendet wurde, dann Einordnung der Vorstufe, die in Schritt D1 verwendet wurde, als Ubergangszustand,

D4 2 wenn dei Wert der Energie dei optimierten Vorstufe (i) oder der Wert der Energie dei optimierten Vorstufe (11) nicht jeweils kleiner ist als der Wert der Energie C3 oder B3 der Vorstufe, die in Schritt D1 verwendet wurde, dann Wiederholung des Verfahrens ab Schritt C

Es wurde uberraschend gefunden, dass die Aufgabe gelost weiden kann, indem durch Einsatz einer geeigneten Monte-Carlo-Methode verschiedene Molekülgeometrien variiert weiden und mit Hilfe eines Gutekriteriums eine Geometrie für einen Ubergangszustand angenahert wird. Des Weiteren wurde uberraschend gefunden, dass die Aufgabe gelost werden kann, indem bei dem Monte-Carloverfahren als Observable der Gradient der Kurve des direkten Reaktionspfades verwendet wird Dadurch lasst sich der zu behandelnde Funktionsraum, der die Molekülgeometrie am Ubergangszustand dei chemischen Reaktion abbilden soll, derart reduzieren, dass die Berechnung von Sattelpunkten ermoglicht wird, ohne zuvor eine Potentialhyperflache zu berechnen oder manuell Molekulgeometrien zu suchen Außerdem wurde überraschend gefunden, dass mit dem erfmdungsgemäßen Verfahren die Berechnung von Sattelpunkten in hochdimensionalen Funktionsraumen moglich wird ohne Newton-Raphson-basierte Methoden zu verwenden Statt der Berechnung von zweiten Ableitungen der Energie nach den Kernkoordinaten wird der Bereich um den Sattelpunkt durch infinitesimale Auslenkungen und anschließende Geometrieoptimierung verifiziert. Dabei muss die Auslenkung entlang der Bindungsdissoziation eine Abnahme der Gesamtenergie ergeben. Auf diese Weise kann die aufwendige Berechnung der zweiten Ableitungen der Energie nach den Kernkoordinaten, wie bei Newton-Raphson-basieiten Verfahren, vollstandig umgangen werden Mit der derzeotigen Computertechnik konnen mit dem eifindungsgemäßen Verfahren deutlich kurzer und schneller auch Ubergangszustande für Molekule mit mehr als 100 Atomen berechnet werden

In **Schritt A** des eifindungsgemaßen Verfahrens wird zunächst eine Startgeometrie erzeugt, indem in Schritt A1 die dreidimensionale Darstellung mindestens eines Molekuls im energetischen Grundzustand bereitgestellt wird Dann wird in Schritt A2 eine Bindung des Molekuls ausgewählt und eine Lange der Bindung, die von der Länge der Bindung im energetischen Grundzustand abweicht, so dass eine Startgeometrie erhalten wird Bevorzugt weist das mindestens eine Molekul aus Schritt A1 eine Große von mehr als 100 Atomen auf, weiter bevorzugt von mehr als 80 Atomen, bevorzugter von mehr als 60 Atomen und/oder betragt die Lange der in Schritt **A2** ausgewahlten Bindung hochstens 230 pm, bevorzugt hochstens 200 pm, weiter bevorzugt hochstens 180 pm, bevorzugter 150 pm

Bevorzugt wird Schritt A durch einen Nutzer ausgefuhrt, der die dreidimensionale Darstellung eines Molekuls im energetischen Grundzustand in Schutt A1, sowie die Auswahl einer Bindung und dei Länge der Bindung in Schritt A2 und die Darstellung der Startgeometrie in Schritt A3 in eine Eingabemaske für das computerimplementierte Verfahren eingibt

In einer weiteren bevorzugten Ausfuhrungsform des erfindungsgemaßen Verfahrens weiden in Schutt **A1** mindestens zwei Molekule I und II bereitgestellt und in Schritt **A2** konnen alternativ oder zusätzlich zu der mindestens einen Bindung auch mindestens ein Abstand zwischen mindestens einem Atom aus Molekul I und mindestens einem Atom aus Molekul II sowie die Lange des mindestens einen Abstands ausgewählt weiden, wobei die Länge des Abstands insbesondere höchstens 230 pm beträgt, bevorzugt hochstens 200 pm, weiter bevorzugt hochstens 180 pm, bevorzugter 150 pm Wenn das Verfahren mit mindestens zwei Molekulen I und II durchgefuhrt wird und die Lange des Abstands zwischen Atomen der verschiedenen Molekule ausgewahlt wird, so kann bevorzugt der Übergangszustand eine Synthesereaktion mit dem Verfahren berechnet werden. Bevorzugt weist Molekül I eine Große von 2 bis 1000 Atomen auf und Molekul II eine Große von 2 bis 1000 Atomen auf, bevorzugter weist Molekul I eine Große von 2 bis 800 Atomen auf und Molekul II eine Große von 2 bis 800 Atomen auf, noch bevorzugter weist Molekül I eine Große von 2 bis 600 Atomen auf und Molekul II eine Größe von 2 bis 600 Atomen auf. Bevorzugt betragt die Summe der Atome aus Molekul I und aus Molekul II mehr als 100 Atome.

Bevorzugt ist Molekül I ein Katalysator für die chemische Reaktion, insbesondere für eine Polymei synthese, und Molekul II ein Edukt der chemischen Reaktion Dabei handelt es sich bei der Polymersynthese in dieser Ausfuhrungsform bevorzugt um Polyurethansynthesen Die chemische Reaktion in dieser Ausfuhrungsform sind bevorzugt auch Synthesen von Monomeren fur Polymerisationsreaktionen, industriell benotigte Grundchemikalien, Additiven, Tensiden und pharmakologischen Wirkstoffen Insbesondere ist die chemische Reaktion eine Synthese fur mit Katalysatoren erhaltene Grundchemikalien oder Edukte fur chemische Synthesen Unter Additiven weiden allgemein Additive für Kunststoffe wie Weichmacher, Antioxidantien, Stellmittel verstanden, sowie Zusatze für Kraftstoffe, bei deren Synthese jeweils Katalysatoren verwendet werden.

In Schritt A3 wird die ausgewahlte Startgeometrie in kartesischen und/oder internen Koordinaten dargestellt Interne Koordinaten beschreiben die räumliche Anoidnung dei Atome relativ zuemander unter Benutzung von Bindungslängen, Bindungswinkel und Torsionswinkel

In **Schritt B** des erfindungsgemaßen Verfahrens wird eine optimierte Startgeometrie ermittelt Dazu wird zunächst in Schritt B1 ein Funktionsraum festgelegt. Der Funktionsraum umfasst die Raumkoordinaten von ausgewählten Atomen, wobei die Raumkoordinaten im Vektorraum aller im Molekül beinhalteten Atomkoordinaten einen Unterraum aufspannen Die ausgewählten Atome für den Funktionsraum sind ein Satz an Atomen, die an einer Bindungsdissoziation beteiligt sind oder bevorzugt an einer Synthesereaktion Des Weiteren umfasst der Funktionsraum die mindestens eine Bindung aus Schritt A2 Die Bei der bevorzugten Ausführungsform mit mindestens zwei Molekulen I und II umfasst dei Funktionsraum den Abstand zwischen mindestens einem Atom aus Molekül I und mindestens einem Atom aus Molekül II

Im folgenden Schritt B2 wird die Startgeometrie einer Geometrieoptimierung mittels einer quantenchemischen Methode unterzogen mit der Randbedingung, dass die in Schritt A2 ausgewählte Lange dei mindestens einen Bindung konstant gehalten wird, so dass eine optimierte Startgeometrie erhalten wird. Dabei umfasst die Geometrieoptimierung alle Atome des als Startgeometrie ausgewählten Molekuls Bei der bevorzugten Ausfuhrungsform mit mindestens zwei Molekülen I und II wird bei der Geometrieoptimierung dei Abstand zwischen mindestens einem Atom aus Molekul I und mindestens einem Atom aus Molekul II konstant gehalten, so dass eine optimierte Startgeometrie ei halten wird

Bei dei Geometrieoptimierung mit Randbedingung wird die Gesamtenergie des Moleküls als Funktion der Kernkoordinaten dei im Molekül enthaltenen Atome mit einem quantenchemischen Verfahren minimiert Um ein lokales Energieminimum zu erhalten wird die Energie nach Gradienten minimiert (zb Steepest Descent), wobei die Energie soweit minimiert wird, wie die Randbedingung dies zulasst

In Schritt B3 wird die Gradient-Norm B3 für die zuvor erhaltene optimierte Startgeometrie ermittelt, wobei die Gradient-Norm durch die erste Ableitung einer Funktion E = f(x) mittels dei quantenchemischen Methode erhalten wird, mit E=Gesamtenergie der optimierten Startgeometrie und x=Kernkoordinaten des Moleküls in der optimierten Startgeometrie. In Schritt B3 wird die gleiche quantenchemische Methode verwendet wie in Schritt B2 Die euklidische Norm dieses Vektors ist die Gradient-Norm Mit anderen Worten wird dei Gradient-Vektor der Energie berechnet Der Gradient-Vektor spannt denselben vektoriellen Raum auf wie das Molekul Jede Komponente des Vektors besteht aus der partiellen

$$\left( \frac{\partial}{\partial x_i} E(x) \right)$$

Ableitung der Energie in dei $x_1$-Koordinate und spannt sich in die Richtung des Einheitsvektors der $x_1$-Koordinate Danach wird die (euklidische) Norm dieses Vektors ermittelt.

Die weitere Fortführung des Verfahrens von der Große der erhaltenen Gradient-Norm B3 ab Sofern die Gradient-Norm B3 $\nabla$ $0 \leq \nabla \leq 0,03$ $E_h$ $a_0^{-1}$ betragt, dann kann die optimierten Startgeometrie als eine Vorstufe für den Ubergangszustand der chemischen Reaktion eingeordnet werden und Schritt C des Verfahrens ausgelassen werden und das Verfahren mit Schritt D1 fortgeführt werden Beträgt die Gradient-Norm B3 $\nabla > 0,03$ $E_h$ $a_0^{-1}$, dann wird Schritt C des Verfahrens durchgefuhrt und zunächst eine Voi stufe fur den Ubergangszustand der chemischen Reaktion ermittelt und zwar ausgehend von der optimierten Startgeometrie Dabei steht $E_h$ fur die Hartree Energie, wobei 1 $E_h$ = 4 3597 · 10⁻¹⁸ J, und $a_0$ steht fur den Bohr-Radius, wobei I $a_0$ = 5.29 · 10⁻¹¹ m

In **Schritt C** wild die Vorstufe für den Ubergangszustand dei chemischen Reaktion ermittelt. Dazu wird die optimierte Startgeometrie unter Anwendung eines Monte-Carlo-Algorithmus variiert indem zufällig generierte Auslenkungen von Atomen in einem Ensemble, das aus einem Molekul oder bevorzugt einer Geometrie von mindestens zwei Molekulen I und II besteht, vorgenommen

Monte-Carlo-Algonthmen oder -Verfahren sind Simulationsverfahren, die analytisch nicht oder nur schwer lösbare mathematische Probleme durch nummerische Naherungen lösen Dabei wird ein wahrschemlichster Verlauf eines Experiments durch eine Vielzahl an zufällig angeordneten Einzelexperimenten beschrieben

In dem erfindungsgemaßen in Schritt C angewandten Monte-Carlo-Algorithmus weiden zufällig generierte Auslenkungen von Atomen in einem Ensemble, das aus einem Molekul oder einer Geometrie von mindestens zwei Molekulen I und II besteht, vorgenommen und die Anderung einer Observablen beobachtet Bei dieser Observablen handelt es sich um eine Gradient-Norm Dabei variiert das erfindungsgemäße Verfahren nui einen Unterraum dei deutlich niedriger dimensional ist als der gesamte Funktionsraum des Ensembles und betrachtet die Anderung der Observablen des gesamten Ensembles als Funktion des reduzierten Funktionsraums

Dazu werden aus dem in Schritt B1 ausgewahlten Funktionsraum, in Schritt C 1 1 mindestens ein Atom ausgewahlt Dies geschieht mit Hilfe einer Zufallszahl Z1 Die Richtung einer Auslenkung des durch Z1 gewahlten Atoms, bzw. die Richtung des Vektors für die Auslenkung, wird in Schritt C1.2 durch eine weitere Zufallszahl Z2 ausgewahlt Die Richtung wird bevorzugt definiert, indem im bevorzugten Schritt C1 2a die Position eines anderen Atoms als dem in Schritt C1 1 ausgewahlten aus dem reduzierten Funktionsraum ausgewahlt wird und dann im bevorzugten Schutt C1 2b die Richtung der Auslenkung ausgewahlt wird, wobei die Position des in Schritt C1 2a ausgewählten Atoms die Richtung des Vektors bestimmt Die Amplitude der Auslenkung wird bevorzugt durch eine dritte Zufallszahl Z3 im bevorzugten Schritt C1.2c bestimmt

In einer bevorzugten Ausfuhrungsform des erfindungsgemäßen Verfahrens umfasst Schritt **C1.2** die weiteren folgenden Schritte

C1 2a Bestimmung eines weiteren Atoms aus dem in Schritt B1 ausgewahlten Funktionsraum, das nicht mit dem in Schritt C1 1 ausgewählten Atom ubereinstimmt,
C1 2b Auswahl dei Richtung des Vektors, wobei die Position des in Schritt C1.2 a ausgewählten Atoms die Richtung des Vektors bestimmt,
C1.2 c zufällige Auswahl dei Lange des Vektors, wobei dei Wert fui die Lange des Vektors positive oder negative Werte annehmen kann

Bevorzugt erfolgt die Gewichtung des zufällig ausgewahlten Betrags des Vektors in Schritt C1 2 durch Multiplikation des Zahlenwerts des zufällig ausgewählten Betrags des Vektors mit dem Zahlenwert dei Gradient-Norm B3

Dadurch dass die Position des in Schritt C1 2 ausgewahlten Atoms die Richtung des Vektors bestimmt, wird der Funktionsraum moglichst stark beschränkt und diese Vorgehensweise führt auch dazu das vorrangig Bindungslangen variiert werden, die Längen aufweisen wie sie sehr wahrscheinlich tatsachlich wahrend einer Reaktion erreicht werden

In Schritt C1 3 wird das in Schritt C 1.1 ausgewahlte Atom anhand des Vektors aus Schritt 1.2 aus seiner Position in dei optimierten Startgeometrie ausgelenkt, so dass eine Voi stufe für den Ubergangszustand der chemischen Reaktion erhalten wird

Die erhaltene Voi stufe fur den Ubergangszustand wird in Schritt C2 einer Geometrieoptimierung mittels der quantenchemischen Methode unterzogen mit dei Randbedingung, dass die mindestens eine Bindung aus Schritt A2 die Lange hat, die in Schritt C1.3 ermittelt wurde In Schutt C2 wird die gleiche quantenchemische Methode verwendet wie in den Schritten B2 und B3. Dann wird in Schritt C3 die Gradient-Norm C3 für die Vorstufe für den Ubergangszustand aus Schritt C2 ermittelt, wobei die Gradient-Norm durch die erste Ableitung der Funktion E = f(x) mittels der quantenchemischen Methode erhalten wird, mit E=Gesamtenergie der Vorstufe für den Ubergangszustand und x=Kernkoordinaten des Molekuls in der Voistufe fur den Ubeigangszustand. In Schritt C3 wird die gleiche quantenchemische Methode verwendet wie in den Schritten B2, B3 und C2

Die weitere Fortführung des Verfahrens hangt von der Große der erhaltenen Gradient-Norm C3 ab Sofern die Gradient-Norm C3 $\nabla$ $0 \leq \nabla \leq 0,03$ $E_h$ $a_0^{-1}$ beträgt, dann kann Schritt D1 des Verfahrens mit der Vorstufe für den Ubergangszustand durchgeführt werden Dabei steht $E_h$ für die Hartree Energie, wobei 1 $E_h$ = 4 3597 $10^{-18}$ J, und $a_0$ steht für den Bohr-Radius, wobei 1 $a_0$ = 5 29 · $10^{-11}$ m

Betragt die Gradient-Norm C3 $\nabla$ $> 0,03$ $E_h$ $a_0^{-1}$, dann weiden in Schritt 4 2 die Schritte C1 bis C3 wiederholt bis eine Gradient-Norm C3 $\nabla$ $0 \leq \nabla \leq 0,03$ $E_h$ $a_0^{-1}$ erhalten wird Durch die Wiederholung der Schritte C1 bis C3 wird ein iteratives Verfahren dargestellt, mit dem die Gradient-Noim minimiert weiden soll Daher soll als Ausgangspunkt für die Wiederholung des Verfahrens jeweils eine Molekulgeometrie gewahlt weiden, die bereits eine moglichst niedrige Gradient-Norm aufweist Bei dei Wiederholung der Schritte C1 bis C3 kann also in Schritt C1 nicht nui die optimierte Startgeometrie variiert weiden, sondern auch eine Voistufe für den Ubeigangszustand, sofern der Wert ihrer Gradient-Norm C3 kleiner ist als derjenige dei optimierten Startgeometrie Bevorzugt weiden die bei der Durchführung des Verfahrens erhaltene optimierte Startgeometrie und erhaltene(n) Vorstufe(n) für den Ubergangszustand sowie die Werte der jeweiligen Gradient-Normen C3 gespeichert

In dem Fall, dass die Schritte C1 bis C3 ein Mal durchgefuhrt wurden, kann aus zwei Molekulgeometrien zui Durchführung der Wiederholung diejenige mit dei niedrigsten Gradient-Norm ausgewählt weiden, namlich entweder die optimierte Startgeometrie oder die in eisten Durchgang erhaltene Vorstufe für den Ubergangszustand In dem Fall, dass die Schritte C1 bis C3 ein Mal durchgeführt wurden, wird in Schritt C1 die Geometrie der Vorstufe für den Übergangszustand variiert, wenn der Wert ihrer Gradient-Norm C3 niedriger ist als der Wert der Gradient-Norm B3 dei optimierten Startgeometrie.

In dem Fall, dass die Schritte C1 bis C3 schon durchgeführt wurden, kann aus mehr als zwei Molekülgeometrien zur Durchführung der Wiederholung diejenige mit der niedrigsten Gradient-Norm ausgewählt werden, namlich entweder die optimierte Startgeometrie oder die in bei den Wiederholungen eihaltenen (mehr als eine) Vorstufen für den Ubergangszustand In dem Fall dass die Schirtte C1 bis C3 mehr als ein Mal durchgefuhrt wurden, wird in Schritt C1 die Geometrie dei optimierten Startgeometrie oder derjenigen Vorstufe für den Ubeigangszustand aus den von angegangen Wiederholungen variiert wird, welche im Vergleich zu allen bisher erhaltenen Gradient-Normen C3 und B3 den niedrigsten Wert für die Gradient-Norm C3 oder B3 aufweist

Bevorzugt ist in Schritt **C4.1** die Gradient-Norm C3 $\leq 0,02$ $E_h$ $a_0^{-1}$, bevorzugter C3 $\leq 0,01$ $E_h$ $a_0^{-1}$, und Schritt **C4.2** wird so lange durchgeführt, bis eine Gradient-Norm C3 $\leq 0,02$ $E_h$ $a_0^{-1}$, bevorzugter C3 $\leq 0,01$ $E_h$ $a_0^{-1}$, erhalten wild

Bevorzugt wird Schritt **C4.2** hochstens 50 Mal, bevorzugt hochstens 40 Mal, bevorzugter hochstens 30 Mal, wiederholt Bevorzugt kann bei der Durchfuhrung von Schritt **C4.2** bei der Wiederholung von Schritt **C1** ern anderes Atom ausgewahlt werden als bei dei von angegangenen Durchführung des Verfahrens.

Ein Vorteil des erfindungsgemäßen Verfahrens ist, dass das bei dei Variation unter Anwendung eines Monte-Carlo-Algorithmus nur die Atomkoordinaten des ausgewahlten Funktionsraums, also des Untenaums der durch die ausgewahlten, an der betrachteten Bindungsdissoziation oder bevorzugt Synthesereaktion beteiligten Atome im Vektorraum aller im Molekul, oder im Molekulensemble, beinhalteten Atomkoordinaten aufgespannt wird Das heißt, der erfindungsgemäße Monte-Carlo-Algorithmus operiert nui in diesem Unterraum, da seine Funktionen nur hierin definiert sind Damit ist der Unterraum der an der Dissoziation, oder bevorzugten Synthesereaktion, beteiligten Kordinaten der Funktionsraum des Monte-Carlo-Algorithmus. Die Berechnung dei Energien und der Gradient-Vektoren (bzw deren Norm) hangen zwar von den gesamten Koordinaten im Molekül ab, jedoch betrachtet dei erfindungsgemäße Monte-Carlo-Algorithmus diese nur in Abhangigkeit der Koordinaten des Unteiraumes Damit bei dieser Betrachtung der Funktionswert, die zu minimierende Gradient-Norm, eindeutig bleibt, wird vor jeder Betrachtung des Funktionswerts die gesamte Molekulstraktur durch eine Geometrieoptimierung mit Randbedingung(en) relaxiert Die Randbedingung(en) sind dabei die durch den Monte-Carlo-Algorithmus eizeugten Bindungsabstande.

In den Schritten **B2, B3, C2, C3,** wird jeweils dieselbe quantenchemische Methode verwendet Bevorzugt ist die quantenchemische Methode aus Schritten **B2, B3, C2, C3, D1** und **D2** eine semiempirische Methode, Dichte-Funktional-theoretische Methode odei eine Annaherung der Schrodinger-Gleichung, insbesondere sind Dichte-Funktional-theoretische Methoden bevorzugt, wie beispielsweise das TPSS-Dichtefunktional mit einem def2-SVP-Basissatz, so wie standardmaßig im Turbomole-Programmpaket implementiert. In einer bevoizugten Ausfuhrungsform weiden die quantenmechanischen Berechnungen mit dem Programmpaket TURBOMOLE durchgeführt Bevorzugt wird zur Berechnung ein Computer mit 16 Core-Prozessoren mit einer Taktfiequenz von 3.20GHz und 25MB Zwischenspeicher mit einem RAM Speichel von 128 GB DDR4 2400 ig ECC verwendet

Bevorzugt ist die chemische Reaktion eine Synthese ausgewählt aus der Gruppe bestehend aus Polymersynthesen, insbesondere Polyurethansynthesen, Synthesen von Monomeren für Polymerisationsreaktionen, industriell benotigte Grundchemikalien, Additiven, Tensiden und pharmakologischen Wirkstoffen Insbesondere ist die chemische Reaktion eine Synthese fur mit Katalysatoren erhaltene Grundchemikalien oder Edukte für chemische Synthesen. Unter Additiven werden allgemern Additive für Kunststoffe wie Weichmacher, Antioxidantien, Stellmittel verstanden, sowie Zusatze für Kraftstoffe, bei deren Synthese jeweils Katalysatoren verwendet werden.

Wenn bei der bevorzugten Ausführungsform des Verfahrens mindestens zwei Molekule I und II berertgestellt werden, konnen bevorzugt die unter den Buchstaben **A, B,** und **C** des Verfahrens genannten Schritte wiederholt weiden, wobei bei jeder Wiederholung im Vergleich zur vorrangegangenen Durchführungen des Verfahrens

Molekül I verandert oder ein anderes Molekul als Molekul I bereitgestellt wird, und
Molekül II nicht verandert und auch kein anderes Molekul als Molekül II beriztgestellt wird,

und der zusätzliche Schritt D0 durchgeführt wird.
D0 Vergleich dei bei den Wiederholungen erhaltenen Gradienten-Norm C3 für die verschiedenen Voi stufen für den Ubergangszustand und Auswahl der Vorstufe fur den Ubergangszustand mit der niedrigsten Gradienten-Noim C3 und Durchführung von Schritt D1 und/odei D2 mit dei ausgewählten Vorstufe für den Ubergangszustand

Bei dieser zuvor beschriebenen bevorzugten Ausführungsform des Verfahrens werden zunächst für unterschiedliche Kombinationen von Molekülen die Gradient-Norm C3 berechnet und die Ergebnisse gespeichert. Dann können in einem bevoizugten Schritt D0 die erhaltenen Werte für die Gradient-Norm C3 verglichen werden und die Kombination von Molekülen mit der niedirgsten Gradienten-Norm C3 ausgewählt weiden Diese bevorzugte Ausfuhrungsform des Verfahrens ermoglicht also den duekten Vergleich von unterschiedlichen Kombinationen von Molekulen

Ein weiterer Gegenstand der Erfindung ist ein System zur Datenverararbertung umfassend Mittel zur Ausführung eines erfindungsgemaßen Verfahrens

Daruber hinaus sind weitere Gegenstande dei Erfindung ein Computerprogramm und ein computerlesbares Sperchermedium, umfassend Befehle, die bei der Ausführung des Programms durch einen Computer diesen veranlassen, die folgende Schritte eines Verfahrens durchzufuhren

**Optional A Erzeugen einer Startgeometrie**

A1 Bereitstellung der dierdimensionalen Darstellung mindestens eines Molekuls im energetischen Grundzustand,
A2 Auswahl von mindestens einer Bindung des mindestens einen Moleküls und Auswahl einer Lange der Bindung, wobei die ausgewählte Länge nicht der Länge der Bindung im energetischen Grundzustand des Molekuls entspricht, so dass eine Startgeometrie für die chemische Reaktion ei halten wird,
A3 Dreidimensionale Darstellung der Startgeometrie in kartesischen und/oder internen Koordinaten,

**B Ermittlung einer optimierten Startgeometrie**

B1 Festlegung eines Funktionsiaumes welcher die mindestens eine Bindung aus Schritt A2 und die durch diese Bindung veibundenen Atome umfasst,
B2 Geometrieoptimierung des in Schritt B1 ausgewahlten Funktionsiaums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,
B3 Ermittlung der Gradient-Norm B3 fur die optimierte Startgeometrie, wobei die Gradient-Norm durch die eiste Ableitung einer Funktion E = f(x) dei quantenchemischen Methode und erhalten wird, mit E=Gesamteneirgie der optimierten Startgeometrie und x=Kernkoordinaten des Molekuls in der optimierten Startgeometrie,
B4.1 wenn die Gradient-Noim B3 $\nabla$ $0 \leq \nabla \leq 0,03$ $E_h$ $a_0^{-1}$ betragt, dann Einordnung der optimierten Startgeometrie als eine Vorstufe für den Ubergangszustand dei chemischen Reaktion und Fortführung des Verfahrens mit Schutt D1, oder
B4 2 wenn die Gradient-Norm B3 $\nabla$ $> 0,03$ $E_h$ $a_0^{-1}$ betragt, dann Ermittlung der Vorstufe fur den Ubeigangszustand der chemischen Reaktion ausgehend von der optimierten Startgeometrie durch ein Verfahren umfassend die folgenden Schirtte

**C Ermittlung der Vorstufe für den Übergangszustand der chemischen Reaktion**

C1 Variation dei optimierten Startgeometrie unter Anwendung eines Monte-Cailo-Algorithmus wobei

C11 wenigstens ein Atom aus dem in Schritt B1 ausgewahlten Funktionsiaum zufällig ausgewahlt wird,

C12 ein Vektor fur eine Auslenkung des in Schritt C1 gewahlten Atoms zufällig ausgewahlt wird, wobei dei zufällig ausgewählte Betrag des Vektors mit dei Gradient-Norm B3 gewichtet wird,

C13 das in Schritt C1.1 ausgewählte Atom anhand des Vektors aus Schritt C12 aus dei Position des Atoms in der optimierten Startgeometrie ausgelenkt wird, so dass eine Vorstufe für einen Ubergangszustand der chemischen Reaktion erhalten wird,

C2 Geometrieoptimierung dei Vorstufe für den Ubergangszustand mittels der quantenchemischen Methode und mit der Randbedingung, dass die mindestens eine Bindung aus Schritt A2 die Lange hat, die sie in der Schutt C3 ermittelt wurde,

C3 Ermittlung dei Gradient-Norm C3 fur die Voistufe fur den Übergangszustand aus Schritt C4, wobei die Gradient-Norm durch die eiste Ableitung der Funktion E = f(x) der quantenchemischen Methode und erhalten wird, mit E=Gesamteneigie der Vorstufe für den Übergangszustand und x=Kernkoordinaten des Moleküls in der Vorstufe für den Ubeigangszustand,

C41 wenn die Gradient-Norm C3 $\nabla$ $0 \leq \nabla \leq 0,03$ $E_h$ $a_0^{-1}$ betragt, dann Fortführung des Verfahrens mit Schritt D1, oder

C4 2 wenn die Gradient-Norm C3 $\nabla > 0,03$ $E_h$ $a_0^{-1}$ betragt, dann Wiederholung dei Schritte C1 bis C3 bis eine Gradient-Norm C3 $\nabla$ $0 \leq \nabla \leq 0,03$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Ubergangszustand variiert wird, wenn der Wert ihrei Gradient-Norm C3 niedriger ist als der Wert der Gradient-Norm B3 der optimierten Startgeometrie, oder

(b) fur den Fall dass die Schritte C1 bis C3 mehi als ein Mal durchgeführt wurden, in Schritt C1 die Geometrie der optimierten Startgeometrie oder derjenigen Vorstufe für den Ubergangszustand aus den von angegangen Wiederholungen variiert wird, welche im Vergleich zu allen bisher erhaltenen Gradient-Normen C3 und B3 den niedrigsten Wert fur die Gradient-Norm C3 oder B3 aufweist,

**D Ermittlung des Übergangszustandes**

D1 Variation der Vorstufe aus Schritt C4 1 oder der Vorstufe aus Schutt B4.1, indem folgende Schritte für ein Atom innerhalb des in B1 definierten Funktionsraums durchgeführt weiden

D1 1 Auswahl eines Atoms aus dem in Schritt B1 ausgewählten Funktionsraum,

D1 2 Auswahl eines Vektors für eine Auslenkung des in Schritt D11 gewählten Atoms

D1 3 Auslenkung des in Schritt D1.1 ausgewählten Atoms anhand des Vektors aus Schritt D1 2 aus seiner Position in der Vorstufe, wobei die Position einmal um einen positiven Wert des Betrags des Vektors und einmal um einen negativen Wert des Betrags des Vektors ausgelenkt wird, so dass bei der Durchfuhrung dei Schritte D1 1 bis D 1.3 zwei ausgelenkte Vorstufen erhalten werden,

D2 Geometrieoptimierung dei zwei ausgelenkten Vorstufen aus Schritt D1 mittels dei quantenchemischen Methode unter dei Randbedingung dass die in D1 erhaltenen Bmdungsabstande konstant gehalten werden, so dass zwei optimierte Vorstufen (1) und (11) erhalten weiden,

D3 Berechnung der Energie dei zwei optimierten Vorstufen (i) und (ii) aus Schritt D2 mittels des quantenchemischen Verfahrens,

D4 Vergleich des Wertes dei Energie der zwei optimierten Voistufen mit dem Wert dei Energie C3 oder B3 dei Vorstufe, die in Schritt D1 verwendet wurde,

D4 1 wenn dei Wert dei Energie der optimierten Vorstufe (i) und der Weit der Energie dei optimierten Vorstufe (ii) kleiner sind als dei Wert der Energie C3 oder B3 der Vorstufe, die in Schritt D1 verwendet wurde, dann Ernordnung dei Vorstufe, die in Schritt D1 verwendet wurde, als Übergangszustand,

D4.2. wenn dei Wert der Energie dei optimierten Vorstufe (i) oder der Wert dei Energie dei optimierten Vorstufe (ii) nicht jeweils kleiner ist als der Wert dei Energie C3 oder B3 der Vorstufe, die in Schritt D1 verwendet wurde, dann Wiederholung des Verfahrens ab Schritt C.

Bevorzugt umfasst das Computerprogramm und/oder das computerlesbare Sperchermedium Befehle, die bei der Ausführung des Programms durch einen Computer diesen veranlassen, die Schritte B bis D des Verfahrens durchzufuhren

Des Weiteren ist die Erfindung auf die Verwendung des eifindungsgemäßen Computerprogramms oder des eifindungsgemaßen computerlesbaren Speichermediums zur Bewertung von Ubergangszuständen einer chemischen Reaktion, insbesondere einer Polymersynthese gerichtet

Die Erfindung betrifft insbesondere die folgenden Ausführungsformen.

In einer eisten Ausführungsform betrifft die Erfindung ein computerimplementiertes Verfahren zur Berechnung von Ubergangszustanden einer chemischen Reaktion umfassend die Schritte

**A Erzeugen einer Startgeometrie**

A1 Bereitstellung der dierdimensionalen Darstellung mindestens eines Moleküls im energetischen Grundzustand,

A2 Auswahl von mindestens einer Bindung des mindestens einen Moleküls und Auswahl einer Lange der Bindung, wobei die ausgewählte Lange nicht dei Lange dei Bindung im energetischen Grundzustand des Moleküls entspricht, so dass eine Startgeometrie fur die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung veibundenen Atome umfasst,

B2 Geometrieoptimierung des in Schritt B1 ausgewahlten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung dei Gradient-Norm B3 fur die optimierte Startgeometrie, wobei die Gradient-Norm durch die erste Ableitung einer Funktion E = f(x) der quantenchemischen Methode und erhalten wird, mit E=Gesamtenergie der optimierten Startgeometrie und x=Kernkoordinaten des Moleculs in der optimierten Startgeometrie,

B4 1 wenn die Gradient-Norm B3 $\nabla$ $0 \leq \nabla \leq 0{,}03$ $E_h$ $a_0^{-1}$ betragt, dann Einordnung der optimierten Startgeometrie als eine Voi stufe für den Ubergangszustand der chemischen Reaktion und Fortführung des Verfahrens mit Schritt D1, oder

B4 2 wenn die Gradient-Noim B3 $\nabla > 0{,}03$ $E_h$ $a_0^{-1}$ beträgt, dann Ermittlung der Vorstufe für den Ubergangszustand der chemischen Reaktion ausgehend von dei optimierten Startgeometrie durch ein Verfahren umfassend die folgenden Schritte.

**C Ermittlung der Vorstufe für den Übergangszustand der chemischen Reaktion**

C1 Variation der optimierten Startgeometrie unter Anwendung eines Monte-Cailo-Algorithmus wobei

C1.1 wenigstens ein Atom aus dem in Schritt B1 ausgewählten Funktionsiaum zufalhg ausgewahlt wird,

C1 2 ein Vektor fur eine Auslenkung des in Schritt C1 gewahlten Atoms zufällig ausgewählt wird, wobei dei zufallig ausgewählte Betrag des Vektors mit der Gradient-Norm B3 gewichtet wird,

C1.3 das in Schritt C1.1 ausgewählte Atom anhand des Vektors aus Schritt C1 2 aus der Position des Atoms in der optimierten Startgeometrie ausgelenkt wird, so dass eine Vorstufe für einen Ubeigangszustand der chemischen Reaktion erhalten wird,

C2 Geometrieoptimierung dei Vorstufe für den Übergangszustand mittels der quantenchemischen Methode und mit der Randbedingung, dass die mindestens eine Bindung aus Schritt A2 die Lange hat, die sie in der Schritt C3 ermittelt wurde,

C3 Ermittlung der Gradient-Norm C3 für die Vorstufe für den Ubergangszustand aus Schritt C4, wobei die Gradient-Norm durch die erste Ableitung dei Funktion E = f(x) der quantenchemischen Methode und erhalten wird, mit E=Gesamtenergie der Vorstufe für den Ubergangszustand und x=Kernkoordinaten des Moleküls in der Vorstufe für den Ubeigangszustand,

C4 1 wenn die Gradient-Norm C3 $\nabla$ $0 \leq \nabla \leq 0{,}03$ $E_h$ $a_0^{-1}$ beträgt, dann Fortführung des Verfahrens mit Schritt D1, oder

C4 2 wenn die Gradient-Norm C3 $\nabla > 0{,}03$ $E_h$ $a_0^{-1}$ beträgt, dann Wiederholung der Schritte C1 bis C3 bis eine Gradient-Norm C3 $\nabla$ $0 \leq \nabla \leq 0{,}03$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrei Gradient-Norm C3 niedriger ist als dei Wert der Gradient-Norm B3 der optimierten Startgeometrie, oder

(b) fur den Fall dass die Schirtte C1 bis C3 mehr als ein Mal durchgeführt wurden, in Schritt C1 die Geometrie der optimierten Startgeometrie oder derjenigen Vorstufe für den Ubergangszustand aus den vorrangegangen Wiederholungen variiert wird, welche im Vergleich zu allen bisher eihaltenen Gradient-Normen C3 und B3 den niedrigsten Wert fur die Gradient-Norm C3 oder B3 aufweist,

**D Ermittlung des Übergangszustandes**

D1 Variation dei Vorstufe aus Schritt C4.1 oder der Vorstufe aus Schritt B4 1, indem folgende Schritte für ein Atom innerhalb des in B1 definierten Funktionsraums durchgeführt weiden

D1.1 Auswahl eines Atoms aus dem in Schritt B1 ausgewählten Funktionsraum,
D1 2 Auswahl eines Vektors für eine Auslenkung des in Schritt D1.1 gewahlten Atoms
D1 3 Auslenkung des in Schritt D1 1 ausgewahlten Atoms anhand des Vektors aus Schritt D1.2 aus seiner Position in der Vorstufe, wobei die Position einmal um einen positiven Wert des Betrags des Vektors und einmal um einen negativen Wert des Betrags des Vektors ausgelenkt wird, so dass bei der Durchführung dei Schritte D1 1 bis D 1 3 zwei ausgelenkte Vorstufen erhalten werden,

D2 Geometrieoptimierung der zwei ausgelenkten Vorstufen aus Schritt D1 mittels der quantenchemischen Methode unter der Randbedingung dass die in D1 erhaltenen Bindungsabstande konstant gehalten weiden, so dass zwei optimierte Vorstufen (i) und (ii) erhalten werden,
D3 Berechnung dei Energie der zwei optimierten Vorstufen (i) und (ii) aus Schritt D2 mittels des quantenchemischen Verfarens,
D4 Vergleich des Weites der Energie der zwei optimierten Vorstufen mit dem Wert der Energie C3 oder B3 dei Vorstufe, die in Schritt D1 verwendet wurde,

D4 1 wenn der Wert dei Energie der optimierten Vorstufe (i) und der Weit der Energie der optimierten Vorstufe (ii) kleiner sind als dei Wert der Energie C3 oder B3 dei Vorstufe, die in Schritt D1 verwendet wurde, dann Einordnung der Vorstufe, die in Schutt D1 verwendet wuide, als Übergangszustand,
D42 wenn der Wert der Energie der optimierten Vorstufe (1) oder dei Wert der Energie dei optimierten Vorstufe (11) nicht jeweils kleiner ist als der Wert der Energie C3 oder B3 der Vorstufe, die in Schritt D1 verwendet wurde, dann Wiederholung des Verfahrens ab Schritt C

In einer zweiten Ausfuhrungsform betrifft die Erfindung ein Vei fahren nach Ausfiihrungsform 1, dadurch gekennzeichnet, dass das mindestens eine Molekul aus Schritt A1 eine Große von mehr als 100 Atomen aufweist und/odei dass die Lange der in Schritt **A2** ausgewahlten Bindung hochstens 230 pm beträgt
In einer dritten Ausführungsform betrifft die Erfindung ein Verfahren nach einer dei Ausführungsformen 1 oder 2, dadurch gekennzeichnet, dass Schritt **C1.2** die weiteren folgenden Schritte umfasst

C12a Bestimmung eines weiteren Atoms aus dem in Schritt B1 ausgewählten Funktionsraum, das nicht mit dem in Schritt C11 ausgewählten Atom ubereinstimmt,
C12b Auswahl dei Richtung des Vektors, wobei die Position des in Schritt C12 a ausgewahlten Atoms die Richtung des Vektors bestimmt,
C12c zufällige Auswahl der Länge des Vektors, wobei der Wert für die Länge des Vektors positive oder negative Weite annehmen kann

In einer vierten Ausführungsform betrifft die Erfindung ein Verfahren nach einer dei vorstehenden Ausführungsformen, dadurch gekennzeichnet, dass die Gewichtung des zufällig ausgewählten Betrags des Vektors in Schritt C12 durch Multiplikation des Zahlenwerts des zufällig ausgewählten Betrags des Vektors mit dem Zahlenwert dei Gradient-Norm B3 eifolgt
In einer fünften Ausführungsform betrifft die Erfindung ein Verfahren nach einer der vorstehenden Ausführungsformen, dadurch gekennzeichnet, dass in Schritt **C4.1** die Gradient-Norm C3 $\leq$ 0,02 $E_h$ $a_0^{-1}$, bevorzugt $\leq$ 0,01 $E_h$ $a_0^{-1}$, ist und Schritt **C4.2** so lange durchgefühit wird, bis eine Gradient-Norm C3 $\leq$ 0,02 $E_h$ $a_0^{-1}$, bevorzugt $\leq$ 0,01 $E_h$ $a_0^{-1}$, erhalten wird
In einer sechsten Ausfuhrungsform betrifft die Erfindung ein Verfahren nach einer dei vorstehenden Ausführungsformen, dadurch gekennzeichnet, dass Schritt **C4.2** hochstens 50 Mal, bevorzugt hochstens 30 Mal, wiederholt wird
In einer siebten Ausfuhrungsform betrifft die Erfindung ein Verfahren nach einer der vorstehenden Ausführungsformen, dadurch gekennzeichnet, dass bei dei Durchführung von Schritt **C4.2** bei der Wiederholung von Schritt **C1** ein anderes Atom ausgewahlt werden kann als bei der von angegangenen Durchführung des Verfahrens
In einer achten Ausführungsform betrifft die Erfindung ein Verfahren nach einer der voi stehenden Ausführungsformen, dadurch gekennzeichnet, dass die quantenchemische Methode aus Schritten **B2, B3, C2, C3** und **D2** eine semiempirische Methode, Dichte-Funktional-theoretische Methode oder eine Annaherung der Schrodinger-Gleichung ist, insbesondere ist die quantenchemische Methode aus Schritten **B2, B3, C2, C3** und **D2** eine Dichte-Funktional-theoietische Methode
In einer neunten Ausführungsform betrifft die Erfindung ein Verfahren nach einer der vorstehenden Ausfuhrungsformen, dadurch gekennzeichnet, dass die chemische Reaktion eine Synthese ist ausgewahlt aus der Gruppe bestehend aus Polymersynthesen, msbesondere Polyurethansynthesen, Synthesen von Monomeien für Polymerisationsreaktionen, industriell benötigte Grundchemikalien Plattformchemikalien, Additiven , Tensiden und pharmakologischen Wirkstoffen
In einer zehnten Ausführungsform betrifft die Erfindung ein Verfahren nach einer der vorstehenden Ausführungsformen, dadurch gekennzeichnet, dass in Schritt **A1** mindestens zwei Molekule I und II bereitgestellt weiden und in Schritt **A2** alternativ oder zusätzlich zu der mindestens einen Bindung auch mindestens ein Abstand zwischen mindestens einem Atom aus Molekül I und mindestens einem Atom aus Molekul II sowie die Lange des mindestens einen Abstands ausgewählt weiden, wobei die Lange des Abstands insbesondere höchstens 230 pm beträgt.

In einer elften Ausführungsform betrifft die Erfindung ein Verfahren nach Ausführungsform 10, dadurch gekennzeichnet, dass die unter den Buchstaben **A, B** und **C** des Verfahrens genannten Schritte wiederholt weiden, wobei bei jeder Wiederholung im Vergleich zur vorrangegangenen Durchführungen des Verfahrens

Molekul I verandert wird oder ein anderes Molekul als Molekul I bereitgestellt wird, und Molekul II nicht verändert wild und auch kein anderes Molekul als Molekul II bereitgestellt wird,

und umfassend den zusatzlichen Schritt

**D0** Vergleich der bei den Wiederholungen erhaltenen Gradienten-Norm C3 für die verschiedenen Vorstufen fur den Ubergangszustand und Auswahl der Vorstufe für den Ubergangszustand mit dei niedrigsten Gradienten-Norm C3 und Durchführung von Schritt D1 und/oder D2 mit der ausgewählten Vorstufe fur den Ubergangszustand

In einer zwolften Ausführungsform betrifft die Erfindung ein Verfahren nach einer dei Ausführungsformen 10 oder 11, dadurch gekennzeichnet, dass Molekul I ein Katalysator für die chemische Reaktion ist, insbesondere für eine Polymersynthese, und Molekul II ein Edukt der chemischen Reaktion

In einer dreizehnten Ausfuhrungsforill betrifft die Erfindung ein Verfahren nach einer der Ausführungsformen 10 bis 12, dadurch gekennzeichnet, dass die Lange des in Schritt **A2** ausgewählten Abstands zwischen mindestens einem Atom aus Molekul I und/oder mindestens einem Atom aus Molekul II insbesondere hochstens 230 pm betragt

In einer viei zehnten Ausführungsform betrifft die Erfindung ein Verfahren nach einer dei Ausführungsformen 10 bis 13, dadurch gekennzeichnet, dass Molekul I eine Große von 2 bis 1000 Atomen aufweist und Molekul II eine Große von 2 bis 1000 Atomen aufweist, bevorzugt soll die Summe der Atome aus Molekul I und aus Molekul II mehr als 100 Atome betragen.

In einer fünfzehnten Ausfuhrungsform betrifft die Erfindung System zur Datenverarbeitung umfassend Mittel zur Ausführung eines Verfahrens umfassend die Schritte

### A Erzeugen einer Startgeometrie

A1 Bereitstellung dei dreidimensionalen Darstellung mindestens eines Moleküls im energetischen Grundzustand,

A2 Auswahl von mindestens einer Bindung des mindestens einen Moleküls und Auswahl einer Lange der Bindung, wobei die ausgewahlte Lange nicht dei Lange dei Bindung im energetischen Grundzustand des Molekuls entspricht, so dass eine Startgeometrie für die chemische Reaktion erhalten wird, A3 Dreidimensionale Darstellung der Startgeometrie in kartesischen und/oder internen Koordinaten,

### B Ermittlung einer optimierten Startgeometrie

B1 Festlegung eines Funktionsraumes welcher die mindestens eine Bindung aus Schritt A2 und die durch diese Bindung verbundenen Atome umfasst, B2 Geometrieoptimierung des in Schritt B1 ausgewahlten Funktionsraums mittels einer quantenchemischen Methode und mit der Randbedingung, dass die in Schritt A2 ausgewählte Lange der mindestens einen Bindung konstant gehalten wird, so dass eine optimierte Startgeometrie erhalten wird, B3 Ermittlung der Gradient-Norm B3 fur die optimierte Startgeometrie, wobei die Gradient-Norm durch die erste Ableitung einer Funktion E = f(x) der quantenchemischen Methode und erhalten wird, mit E=Gesamtenergie der optimierten Startgeometrie und x=Kernkoordinaten des Molekuls in dei optimierten Startgeometrie, B4 1 wenn die Gradient-Norm B3 $\nabla$ $0 \leq \nabla \leq 0{,}03$ $E_h$ $a_0^{-1}$ beträgt, dann Ernordnung der optimierten Startgeometrie als eine Vorstufe für den Ubergangszustand der chemischen Reaktion und Fortführung des Verfahrens mit Schritt D1, oder B4 2 wenn die Gradient-Norm B3 $\nabla > 0{,}03$ $E_h$ $a_0^{-1}$ betragt, dann Ermittlung der Vorstufe für den Ubergangszustand der chemischen Reaktion ausgehend von dei optimierten Startgeometrie durch ein Verfahren umfassend die folgenden Schritte

### C Ermittlung der Vorstufe für den Übergangszustand der chemischen Reaktion

C1 Variation der optimierten Startgeometrie unter Anwendung eines Monte-Carlo-Algorithmus wobei

C11 wenigstens ein Atom aus dem in Schutt B1 ausgewählten Funktionsraum zufällig ausgewahlt wird, C12 ein Vektor für eine Auslenkung des in Schritt C1 gewahlten Atoms zufällig ausgewählt wird, wobei dei zufällig ausgewählte Betrag des Vektors mit der Gradient-Norm B3 gewichtet wird, C1.3 das in Schritt C11 ausgewählte Atom anhand des Vektors aus Schritt C12 aus dei Position des Atoms in dei optimierten Startgeometrie ausgelenkt wird, so dass eine Vorstufe für einen Übergangszustand dei chemischen Reaktion erhalten wird,

C2 Geometrieoptimierung dei Vorstufe für den Ubergangszustand mittels der quantenchemischen Methode und mit der Randbedingung, dass die mindestens eine Bindung aus Schritt A2 die Lange hat, die sie in der Schutt C3 ermittelt wurde,
C3 Ermittlung der Gradient-Norm C3 für die Vorstufe für den Übergangszustand aus Schritt C4, wobei die Gradient-Norm durch die erste Ableitung der Funktion E = f(x) dei quantenchemischen Methode und erhalten wird, mit E=Gesamtenergie dei Vorstufe für den Übergangszustand und x=Kernkoordinaten des Molekuls in der Vorstufe für den Übergangszustand,
C4 1 wenn die Gradient-Norm C3 $\nabla$ $0 \leq \nabla \leq 0,03$ $E_h$ $a_0^{-1}$ betragt, dann Fortführung des Verfahrens mit Schritt D1, oder
C42 wenn die Gradient-Norm C3 $\nabla > 0,03$ $E_h$ $a_0^{-1}$ beträgt, dann Wiederholung dei Schritte C1 bis C3 bis eine Gradient-Norm C3 $\nabla$ $0 \leq \nabla \leq 0,03$ $E_h$ $a_0^{-1}$ erhalten wird, wobei

(a) fui den Fall, dass die Schritte C1 bis C3 ein Mal durchgeführt wurden, in Schritt C1 die Geometrie der Vorstufe für den Ubergangszustand varnert wird, wenn der Wert ihrer Gradient-Norm C3 niedriger ist als dei Wert dei Gradient-Norm B3 der optimierten Startgeometrie, oder
(b) fur den Fall dass die Schritte C1 bis C3 mehr als ein Mal durchgefuhrt wurden, in Schritt C1 die Geometrie dei optimierten Startgeometrie oder derjenigen Vorstufe für den Übergangszustand aus den von angegangen Wiederholungen varnert wird, welche im Vergleich zu allen bisher erhaltenen Gradient-Normen C3 und B3 den niedrigsten Wert fur die Gradient-Norm C3 oder B3 aufweist,

## D Ermittlung des Übergangszustandes

D1 Variation der Vorstufe aus Schritt C4 1 oder dei Vorstufe aus Schritt B4 1, indem folgende Schritte für ein Atom innerhalb des in B1 definierten Funktionsraums durchgeführt werden

D11 Auswahl eines Atoms aus dem in Schritt B1 ausgewahlten Funktionsraum,
D12 Auswahl eines Vektors für eine Auslenkung des in Schritt D11 gewahlten Atoms
D1.3 Auslenkung des in Schritt D1.1 ausgewahlten Atoms anhand des Vektors aus Schritt D12 aus seiner Position in dei Vorstufe, wobei die Position einmal um einen positiven Wert des Betrags des Vektors und einmal um einen negativen Wert des Betrags des Vektors ausgelenkt wird, so dass bei der Durchführung der Schritte D11 bis D13 zwei ausgelenkte Voi stufen erhalten werden,

D2 Geometrieoptimierung dei zwei ausgelenkten Vorstufen aus Schritt D1 mittels der quantenchemischen Methode unter der Randbedingung dass die in D1 erhaltenen Bindungsabstande konstant gehalten weiden, so dass zwei optimierte Vorstufen (1) und (11) erhalten werden,
D3 Berechnung dei Energie der zwei optimierten Vorstufen (1) und (11) aus Schritt D2 mittels des quantenchemischen Verfahrens,
D4 Vergleich des Wertes dei Energie der zwei optimierten Vorstufen mit dem Wert der Energie C3 oder B3 dei Vorstufe, die in Schutt D1 verwendet wurde,

D41 wenn dei Wert dei Energie der optimierten Vorstufe (1) und der Weit der Energie der optimierten Vorstufe (11) kleiner sind als dei Wert dei Energie C3 oder B3 dei Vorstufe, die in Schritt D1 verwendet wurde, dann Einordnung der Vorstufe, die in Schritt D1 verwendet wurde, als Übergangszustand,
D4.2. wenn der Weit dei Energie der optimierten Vorstufe (1) oder der Weit dei Energie der optimierten Vorstufe (11) nicht jeweils kleiner ist als dei Wert der Energie C3 oder B3 der Vorstufe, die in Schritt D1 verwendet wurde, dann Wiederholung des Verfahrens ab Schritt C

In einer sechzehnten Ausführungsform betrifft die Erfindung ein Computerprogramm, umfassend Befehle, die bei der Ausfuhrung des Programms durch einen Computer diesen veianlassen, die folgenden Schritte eines Verfahrens durchzuführen

## Optional A Erzeugen einer Startgeometrie

A1 Bereitstellung der dreidimensionalen Darstellung mindestens eines Molekuls im energetischen Grundzustand,
A2 Auswahl von mindestens einer Bindung des mindestens einen Molekuls und Auswahl einer Lange dei Bindung, wobei die ausgewahlte Lange nicht dei Lange der Bindung im energetischen Grundzustand des Molekuls entspricht, so dass eine Startgeometrie für die chemische Reaktion erhalten wird,
A3 Dreidimensionale Darstellung der Startgeometrie in kai tesischen und/oder internen Koordinaten,

## B Ermittlung einer optimierten Startgeometrie

B1 Festlegung eines Funktionsraumes welcher die mindestens eine Bindung aus Schritt A2 und die durch diese Bindung verbundenen Atome umfasst,
B2 Geometrieoptimierung des in Schritt B1 ausgewahlten Funktionsraums mittels einer quantenchemischen Methode und mit dei Randbedingung, dass die in Schutt A2 ausgewahlte Lange der mindestens einen Bindung konstant gehalten wird, so dass eine optimierte Startgeometrie ei halten wird,
B3 Ermittlung der Gradient-Norm B3 für die optimierte Startgeometrie, wobei die Gradient-Norm durch die erste Ableitung einer Funktion E = f(x) dei quantenchemischen Methode und erhalten wird, mit E=Gesamteneigie der optimierten Startgeometrie und x=Kernkoordinaten des Molekuls in der optimierten Startgeometrie,
B41 wenn die Gradient-Norm B3 $\nabla$ $0 \leq \nabla \leq 0,03$ $E_h$ $a_0^{-1}$ betragt, dann Ernordnung der optimierten Startgeometrie als eine Vorstufe für den Ubergangszustand der chemischen Reaktion und Fortführung des Verfahrens mit Schritt D1, oder
B42 wenn die Gradient-Noim B3 $\nabla$ > 0,03 $E_h$ $a_0^{-1}$ beträgt, dann Ermittlung der Vorstufe fur den Ubergangszustand dei chemischen Reaktion ausgehend von der optimierten Startgeometrie durch ein Verfahren umfassend die folgenden Schritte.

**C Ermittlung der Vorstufe für den Ubergangszustand der chemischen Reaktion**

C1 Variation dei optimierten Startgeometrie unter Anwendung eines Monte-Carlo-Algorithmus wobei

C11 wenigstens ein Atom aus dem in Schritt B1 ausgewahlten Funktionsraum zufällig ausgewahlt wird,
C1.2 ein Vektor fur eine Auslenkung des in Schritt C1 gewahlten Atoms zufällig ausgewahlt wird, wobei dei zufällig ausgewahlte Betrag des Vektors mit der Gradient-Norm B3 gewichtet wird,
C13 das in Schritt C1.1 ausgewahlte Atom anhand des Vektors aus Schritt C12 aus der Position des Atoms in dei optimierten Startgeometrie ausgelenkt wird, so dass eine Vorstufe für einen Ubergangszustand der chemischen Reaktion erhalten wird,

C2 Geometrieoptimierung dei Vorstufe für den Ubergangszustand mittels dei quantenchemischen Methode und mit der Randbedingung, dass die mindestens eine Bindung aus Schritt A2 die Lange hat, die sie in dei Schritt C3 ermittelt wurde,
C3 Ermittlung dei Gradient-Norm C3 für die Vorstufe für den Ubergangszustand aus Schutt C4, wobei die Gradient-Norm durch die erste Ableitung dei Funktion E = f(x) dei quantenchemischen Methode und erhalten wird, mit E=Gesamtenergie der Vorstufe für den Ubergangszustand und x=Kernkoordinaten des Molekuls in der Vorstufe für den Ubergangszustand,
C4 1 wenn die Gradient-Norm C3 $\nabla$ $0 \leq \nabla \leq 0,03$ $E_h$ $a_0^{-1}$ betragt, dann Fortführung des Verfahrens mit Schutt D1, oder
C42 wenn die Gradient-Norm C3 $\nabla$ > 0,03 $E_h$ $a_0^{-1}$ betragt, dann Wiederholung der Schritte C1 bis C3 bis eine Gradient-Norm C3 $\nabla$ $0 \leq \nabla \leq 0,03$ $E_h$ $a_0$-1 erhalten wird, wobei

(a) für den Fall, dass die Schritte C1 bis C3 ein Mal durchgeführt wurden, in Schritt C1 die Geometrie der Vorstufe für den Ubergangszustand varnert wird, wenn der Wert ihrer Gradient-Norm C3 niedriger ist als dei Wert der Gradient-Norm B3 der optimierten Startgeometrie, oder

(b) fur den Fall dass die Schritte C1 bis C3 mehr als ein Mal durchgeführt wurden, in Schritt C1 die Geometrie dei optimierten Startgeometrie oder derjenigen Vorstufe für den Ubergangszustand aus den vorrangegangen Wiederholungen varnert wird, welche im Vei gleich zu allen bisher erhaltenen Gradient-Normen C3 und B3 den medrigsten Wert für die Gradient-Norm C3 oder B3 aufweist,

**D Ermittlung des Übergangszustandes**

D1 Variation der Vorstufe aus Schritt C4 1 oder dei Vorstufe aus Schritt B4 1, indem folgende Schritte für ein Atom innerhalb des in B1 definierten Funktionsraums durchgeführt werden

D11 Auswahl eines Atoms aus dem in Schritt B1 ausgewahlten Funktionsraum,
D12 Auswahl eines Vektors für eine Auslenkung des in Schritt D11 gewahlten Atoms
D1.3 Auslenkung des in Schritt D11 ausgewahlten Atoms anhand des Vektors aus Schritt D12 aus seiner Position in der Vorstufe, wobei die Position einmal um einen positiven Wert des Betrags des Vektors und ernmal um einen negativen Wert des Betrags des Vektors ausgelenkt wird, so dass bei der Durchführung dei Schritte D1.1 bis D 1.3 zwei ausgelenkte Vorstufen erhalten werden,

D2 Geometrieoptimierung der zwei ausgelenkten Vorstufen aus Schritt D1 mittels der quantenchemischen Methode unter dei Randbedingung dass die in D1 erhaltenen Bindungsabstande konstant gehalten weiden, so dass zwei optimierte Vorstufen (1) und (11) erhalten weiden,

D3 Berechnung dei Energie dei zwei optimierten Vorstufen (1) und (11) aus Schritt D3 mittels des quantenchemischen Verfahrens,

D4 Vergleich des Wertes der Energie der zwei optimierten Vorstufen mit dem Wert dei Energie C3 oder B3 dei Vorstufe, die in Schritt D1 verwendet wurde,

D41 wenn der Wert der Energie der optimierten Vorstufe (1) und der Wert dei Energie dei optimierten Vorstufe (11) kleiner sind als der Weit dei Energie C3 oder B3 der Vorstufe, die in Schritt D1 verwendet wurde, dann Ernordnung der Vorstufe, die in Schritt D1 verwendet wurde, als Ubergangszustand,

D42 wenn dei Wert der Energie der optimierten Vorstufe (1) oder dei Wert dei Energie dei optimierten Vorstufe (11) nicht jeweils kleiner ist als der Wert der Energie C3 oder B3 der Vorstufe, die in Schritt D1 verwendet wurde, dann Wiederholung des Verfahrens ab Schritt C

In einer siebzehnten Ausfuhrungsform betrifft die Erfindung ein computerlesbares Sperchermedium, umfassend Befehle, die bei der Ausführung durch einen Computer diesen veranlassen, die folgenden Schritte eines Verfahrens durchzuführen

## Optional A Erzeugen einer Startgeometrie

A1 Bereitstellung der dreidimensionalen Darstellung mindestens eines Molekuls im energetischen Grundzustand,

A2 Auswahl von mindestens erner Bindung des mindestens einen Molekuls und Auswahl einer Länge der Bindung, wobei die ausgewahlte Länge nicht dei Lange dei Bindung im energetischen Grundzustand des Molekuls entspricht, so dass eine Startgeometrie für die chemische Reaktion erhalten wird,

A3 Dreidimensionale Darstellung dei Startgeometrie in kartesischen und/oder internen Koordinaten,

## B Ermittlung einer optimierten Startgeometrie

B1 Festlegung eines Funktionsraumes welcher die mindestens eine Bindung aus Schritt A2 und die durch diese Bindung verbundenen Atome umfasst,

B2 Geometrieoptimierung des in Schritt B1 ausgewahlten Funktionsraums mittels einer quantenchemischen Methode und mit der Randbedingung, dass die in Schritt A2 ausgewählte Länge dei mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) dei quantenchemischen Methode und erhalten wird, mit E=Gesamtenergie der optimierten Startgeometrie und x=Kernkoordinaten des Moleküls in der optimierten Startgeometrie,

B4 1 wenn die Gradient-Norm B3 $\nabla$ $0 \leq \nabla \leq 0{,}03$ $E_h$ $a0^{-1}$ betragt, dann Ernordnung der optimierten Startgeometrie als eine Vorstufe für den Ubergangszustand der chemischen Reaktion und Fortführung des Verfahrens mit Schritt D1, oder

B42 wenn die Gradient-Norm B3 $\nabla$ $> 0{,}03$ $E_h$ $a_0^{-1}$ betragt, dann Ermittlung dei Vorstufe für den Ubergangszustand dei chemischen Reaktion ausgehend von dei optimierten Startgeometrie durch ein Verfahren umfassend die folgenden Schritte

## C Ermittlung der Vorstufe für den Übergangszustand der chemischen Reaktion

C1 Variation der optimierten Startgeometrie unter Anwendung eines Monte-Carlo-Algorithmus wobei

C11 wenigstens ein Atom aus dem in Schritt B1 ausgewählten Funktionsraum zufällig ausgewahlt wird,

C1.2 ein Vektor für eine Auslenkung des in Schritt C1 gewahlten Atoms zufällig ausgewahlt wird, wobei der zufällig ausgewählte Betrag des Vektors mit dei Gradient-Norm B3 gewichtet wird,

C13 das in Schritt C11 ausgewählte Atom anhand des Vektors aus Schritt C12 aus der Position des Atoms in der optimierten Startgeometrie ausgelenkt wird, so dass eine Vorstufe für einen Ubeigangszustand der chemischen Reaktion erhalten wird,

C2 Geometrieoptimierung dei Vorstufe für den Ubergangszustand mittels der quantenchemischen Methode und mit der Randbedingung, dass die mindestens eine Bindung aus Schritt A2 die Lange hat, die sie in der Schritt C3 ermittelt wurde,

C3 Ermittlung der Gradient-Norm C3 für die Vorstufe für den Ubergangszustand aus Schritt C4, wobei die Gradient-Norm durch die erste Ableitung dei Funktion E = f(x) der quantenchemischen Methode und erhalten wird, mit E=Gesamtenergie der Vorstufe für den Ubergangszustand und x=Kernkoordmaten des Molekuls in der Vorstufe für den Ubergangszustand,

C4 1 wenn die Gradient-Norm C3 $\nabla$ $0 \leq \nabla \leq 0{,}03$ $E_h$ $a_0^{-1}$ betragt, dann Fortführung des Verfahrens mit Schritt D1, oder

C42 wenn die Gradient-Norm C3 $\nabla$ $> 0{,}03$ $E_h$ $a_0^{-1}$ betragt, dann Wiederholung der Schritte C1 bis C3 bis eine Gradient-Norm C3 $\nabla$ $0 \leq \nabla \leq 0{,}03$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Ubergangszustand varnert wird, wenn der Wert ihrer Gradient-Norm C3 niedriger ist als der Wert der Gradient-Norm B3 der optimierten Startgeometrie, oder

(b) fur den Fall dass die Schritte C1 bis C3 mehr als ein Mal durchgeführt wurden, in Schritt C1 die Geometrie dei optimierten Startgeometrie oder derjenigen Vorstufe für den Übergangszustand aus den vorrangegangen Wiederholungen varnert wird,

welche im Vergleich zu allen bisher erhaltenen Gradient-Normen C3 und B3 den niedrigsten Wert fur die Gradient-Norm C3 oder B3 aufweist,

**D Ermittlung des Übergangszustandes**

D1 Variation der Vorstufe aus Schritt C4 1 oder dei Vorstufe aus Schritt B4 1, indem folgende Schritte für ein Atom innerhalb des in B1 definierten Funktionsraums durchgeführt werden

D1 1 Auswahl eines Atoms aus dem in Schritt B1 ausgewählten Funktionsraum,
D1.2 Auswahl eines Vektors für eine Auslenkung des in Schritt D1. 1 gewählten Atoms
D1 3 Auslenkung des in Schritt D1.1 ausgewählten Atoms anhand des Vektors aus Schritt D1 2 aus seiner Position in dei Vorstufe, wobei die Position einmal um einen positiven Wert des Betrags des Vektors und einmal um einen negativen Weit des Betrags des Vektors ausgelenkt wird, so dass bei dei Durchführung der Schritte D1 1 bis D1 3 zwei ausgelenkte Vorstufen erhalten weiden,

D2 Geometrieoptimierung dei zwei ausgelenkten Vorstufen aus Schritt D1 mittels der quantenchemischen Methode unter der Randbedingung dass die in D1 erhaltenen Bindungsabstande konstant gehalten weiden, so dass zwei optimierte Vorstufen (i) und (ii) erhalten werden,
D3 Berechnung der Energie der zwei optimierten Vorstufen (i) und (ii) aus Schritt D3 mittels des quantenchemischen Verfahrens,
D4 Vergleich des Wertes der Energie dei zwei optimierten Voi stufen mit dem Wert der Energie C3 oder B3 der Vorstufe, die in Schritt D1 verwendet wurde,

D4 1 wenn der Wert der Energie der optimierten Vorstufe (i) und der Wert der Energie der optimierten Vorstufe (ii) kleiner sind als der Wert der Energie C3 oder B3 der Vorstufe, die in Schritt D1 verwendet wurde, dann Einordnung dei Vorstufe, die in Schritt D1 verwendet wurde, als Ubergangszustand,
D4 2 wenn dei Wert dei Energie dei optimierten Vorstufe (i) oder der Weit der Energie dei optimierten Vorstufe (ii) nicht jeweils kleiner ist als dei Wert der Energie C3 oder B3 der Vorstufe, die in Schritt D1 verwendet wurde, dann Wiederholung des Verfahrens ab Schritt C.

In einer achtzehnten Ausführungsform betrifft die Erfindung die Verwendung eines Computerprogramms nach Ausführungsform 16 oder eines computerlesbaren Speichermediums nach Ausführungsform 17 zur Bewertung von Ubergangszuständen einer chemischen Reaktion, insbesondere einer Polymersynthese.

**Patentansprüche**

1. Computenmplementiertes Verfahren zur Berechnung von Ubergangszustanden einer chemischen Reaktion umfassend die Schritte

   **A Erzeugen einer Startgeometrie**

   A1 Bereitstellung dei dreidimensionalen Darstellung mindestens eines Molekuls im energetischen Grundzustand,
   A2 Auswahl von mindestens einer Bindung des mindestens einen Molekuls und Auswahl einer Länge dei Bindung, wobei die ausgewählte Lange nicht dei Lange dei Bindung im energetischen Grundzustand des Molekuls entspricht, so dass eine Startgeometrie für die chemische Reaktion erhalten wird,
   A3 Dreidimensionale Darstellung der Startgeometrie in kartesischen und/oder internen Koordinaten,

   **B Ermittlung einer optimierten Startgeometrie**

   B1 Festlegung eines Funktionsraumes welcher die mindestens eine Bindung aus Schritt A2 und die durch diese Bindung veibundenen Atome umfasst,
   B2 Geometrieoptimierung des in Schritt B1 ausgewählten Funktionsraums mittels einer quantenchemischen Methode und mit der Randbedingung, dass die in Schritt A2 ausgewählte Lange der mindestens einen Bindung konstant gehalten wird, so dass eine optimierte Startgeometrie ei halten wird,
   B3 Ermittlung dei Gradient-Norm B3 fui die optimierte Startgeometrie, wobei die Gradient-Norm durch die eiste Ableitung einer Funktion E = f(x) dei quantenchemischen Methode und erhalten wird, mit E=Gesamtenergie dei optimierten Startgeometrie und x=Kernkoordinaten des Moleküls in der optimierten Startgeometrie,

B4 1 wenn die Gradient-Noim B3 $\nabla$ $0 \leq \nabla \leq$ 0,03 $E_h\, a_0^{-1}$ betragt, dann Einordnung dei optimierten Startgeometrie als eine Vorstufe für den Übergangszustand der chemischen Reaktion und Fortführung des Verfahrens mit Schritt D1, oder

B4 2 wenn die Gradient-Norm B3 $\nabla$ > 0,03 $E_h\, a_0^{-1}$ betragt, dann Ermittlung der Vorstufe für den Übergangszustand der chemischen Reaktion ausgehend von der optimierten Startgeometrie durch ein Verfahren umfassend die folgenden Schritte

**C Ermittlung der Vorstufe für den Übergangszustand der chemischen Reaktion**

C1 Variation dei optimierten Startgeometrie unter Anwendung eines Monte-Cailo-Algorithmus wobei

C1 1 wenigstens ein Atom aus dem in Schritt B1 ausgewählten Funktionsiaum zufällig ausgewählt wird,

C1 2 ein Vektor für eine Auslenkung des in Schritt C1 gewahlten Atoms zufällig ausgewählt wird, wobei der zufällig ausgewählte Betrag des Vektors mit dei Gradient-Norm B3 gewichtet wird,

C1 3 das in Schritt C1 1 ausgewählte Atom anhand des Vektors aus Schritt C1 2 aus dei Position des Atoms in dei optimierten Startgeometrie ausgelenkt wird, so dass eine Vorstufe für einen Ubergangszustand der chemischen Reaktion erhalten wird,

C2 Geometrieoptimierung dei Vorstufe für den Ubergangszustand mittels der quantenchemischen Methode und mit der Randbedingung, dass die mindestens eine Bindung aus Schritt A2 die Länge hat, die sie in der Schritt C3 ermittelt wurde,

C3 Ermittlung der Gradient-Norm C3 für die Vorstufe für den Übergangszustand aus Schritt C4, wobei die Gradient-Norm durch die erste Ableitung dei Funktion E = f(x) dei quantenchemischen Methode und ei halten wird, mit E=Gesamtenergie der Vorstufe für den Übergangszustand und x=Kernkoordinaten des Moleküls in der Voi stufe für den Ubergangszustand,

C4.1 wenn die Gradient-Norm C3 $\nabla$ $0 \leq \nabla \leq$

0,03 $E_h\, a_0^{-1}$ betragt, dann Fortführung des Verfahrens mit Schritt D1, oder

C4 2 wenn die Gradient-Norm C3 $\nabla$ > 0,03 $E_h\, a_0^{-1}$ betragt, dann Wiederholung dei Schritte C1 bis C3 bis eine Gradient-Norm C3 $\nabla$ $0 \leq \nabla \leq$ 0,03 $E_h\, a_0^{-1}$ erhalten wird, wobei

(a) für den Fall, dass die Schritte C1 bis C3 ein Mal durchgeführt wurden, in Schritt C1 die Geometrie dei Vorstufe für den Ubergangszustand varnert wird, wenn dei Wert ihrer Gradient-Norm C3 niedriger ist als dei Wert der Gradient-Norm B3 dei optimierten Startgeometrie, oder

(b) für den Fall dass die Schritte C1 bis C3 mehr als ein Mal durchgeführt wurden, in Schritt C1 die Geometrie dei optimierten Startgeometrie oder derjenigen Vorstufe für den Ubeigangszustand aus den vorrangegangen Wiederholungen varnert wird, welche im Vergleich zu allen bisher erhaltenen Gradient-Normen C3 und B3 den medrigsten Wert für die Gradient-Norm C3 oder B3 aufweist,

**D Ermittlung des Übergangszustandes**

D1 Variation der Vorstufe aus Schritt C4.1 oder dei Vorstufe aus Schritt B4 1, indem folgende Schritte für ein Atom innerhalb des in B1 definierten Funktionsraums durchgeführt werden

D1 1 Auswahl eines Atoms aus dem in Schritt B1 ausgewählten Funktionsraum,

D1 2 Auswahl eines Vektors für eine Auslenkung des in Schritt D1.1 gewählten Atoms

D1 3 Auslenkung des in Schritt D1 1 ausgewählten Atoms anhand des Vektors aus Schritt D1 2 aus seiner Position in dei Vorstufe, wobei die Position einmal um einen positiven Wert des Betrags des Vektors und einmal um einen negativen Wert des Betrags des Vektors ausgelenkt wird, so dass bei dei Durchführung der Schritte D1 1 bis D 1 3 zwei ausgelenkte Vorstufen erhalten weiden,

D2 Geometrieoptimierung dei zwei ausgelenkten Vorstufen aus Schritt D1 mittels dei quantenchemischen Methode unter der Randbedingung dass die in D1 erhaltenen Bindungsabstande konstant gehalten werden, so dass zwei optimierte Vorstufen (i) und (ii) erhalten werden,

D3 Berechnung der Energie dei zwei optimierten Vorstufen (i) und (ii) aus Schritt D2 mittels des quantenchemischen Verfahrens,

D4 Vergleich des Wertes der Energie dei zwei optimierten Vorstufen mit dem Weit der Energie C3 oder B3 dei Voi stufe, die in Schritt D1 verwendet wurde,

D4 1 wenn der Weit der Energie der optimierten Vorstufe (i) und der Wert der Energie der optimierten Vorstufe (ii) kleiner sind als der Wert der Energie C3 oder B3 dei Vorstufe, die in Schritt D1 verwendet wurde, dann Einordnung der Vorstufe, die in Schritt D1 verwendet wurde, als Übergangszustand,

D4 2 wenn der Wert dei Energie der optimierten Vorstufe (i) oder dei Wert der Energie dei optimierten Vorstufe (ii) nicht jeweils kleiner ist als dei Wert der Energie C3 oder B3 der Vorstufe, die in Schritt D1 verwendet wurde, dann Wiederholung des Verfahrens ab Schritt C

2. Verfahren nach Anspruch 1, **dadurch gekennzeichnet, dass** das mindestens eine Molekül aus Schritt A1 eine Große von mehr als 100 Atomen aufweist und/oder dass die Länge dei in Schritt **A2** ausgewählten Bindung höchstens 230 pm betragt

3. Verfahren nach Anspruch 1 oder 2, **dadurch gekennzeichnet, dass** Schritt **C1.2** die weiteren folgenden Schritte umfasst

C1.2a Bestimmung eines weiteren Atoms aus dem in Schritt B1 ausgewählten Funkctionsraum, das nicht mit dem in Schritt C1 1 ausgewählten Atom übereinstimmt,

C1 2b Auswahl der Richtung des Vektors, wobei die Position des in Schritt C1.2 a ausgewählten Atoms die Richtung des Vektors bestimmt,

C1 2.c zufallige Auswahl der Lange des Vektors, wobei der Weit für die Länge des Vektors positive oder negative Werte annehmen kann

4. Verfahren nach einem der vorstehenden Anspruche, **dadurch gekennzeichnet, dass** die Gewichtung des zufällig ausgewahlten Betrags des Vektors in Schritt C1 2 durch Multiplikation des Zahlenwerts des zufällig ausgewählten Betrags des Vektors mit dem Zahlenwert der Gradient-Norm B3 erfolgt

5. Verfahren nach einem der vorstehenden Anspruche, **dadurch gekennzeichnet, dass** in Schritt **C4.1** die Gradient-Norm C3 $\leq 0,02$ $E_h\, a_0^{-1}$, bevorzugt $\leq 0,01$ $E_h\, a_0^{-1}$, ist und Schritt C4.2 so lange durchgeführt wird, bis eine Gradient-Norm C3 $\leq 0,02$ $E_h\, a_0^{-1}$, bevorzugt $\leq 0,01$ $E_h\, a_0^{-1}$, erhalten wird

6. Verfahren nach einem der vorstehenden Anspruche, **dadurch gekennzeichnet, dass** Schritt **C4.2** höchstens 50 Mal, bevorzugt hochstens 30 Mal, wiederholt wird.

7. Verfahren nach einem der voi stehenden Anspruche, **dadurch gekennzeichnet, dass** bei dei Durchführung von Schritt **C4.2** bei dei Wiederholung von Schritt **C1** ein anderes Atom ausgewählt werden kann als bei der von angegangenen Durchführung des Verfahrens

8. Verfahren nach einem der vorstehenden Ansprüche, **dadurch gekennzeichnet, dass** die quantenchemische Methode aus Schritten **B2, B3, C2, C3** und **D2** eine semiempirische Methode, Dichte-Funktional-theoretische Methode oder eine Annaherung der Schrodinger-Gleichung ist, insbesondere ist die quantenchemische Methode aus Schritten **B2, B3, C2, C3** und **D2** eine Dichte-Funktional-theoretische Methode

9. Verfahren nach einem der vorstehenden Anspruche, **dadurch gekennzeichnet, dass** die chemische Reaktion eine Synthese ist ausgewählt aus der Gruppe bestehend aus Polymersynthesen, insbesondere Polymethansynthesen, Synthesen von Monomeren für Polymerisationsieaktionen, industriell benotigte Grundchemikalien Plattformchemikalien, Additiven , Tensiden und pharmakologischen Wirkstoffen

10. Veifahien nach einem der vorstehenden Anspruche, **dadurch gekennzeichnet, dass** in Schritt **A1** mindestens zwei Moleküle I und II bereitgestellt werden und in Schritt **A2** alternativ oder zusatzlich zu der mindestens einen Bindung auch mindestens ein Abstand zwischen mindestens einem Atom aus Molekül I und mindestens einem Atom aus Molekul II sowie die Lange des mindestens einen Abstands ausgewählt weiden, wobei die Lange des Abstands insbesondere höchstens 230 pm betragt

11. Verfahren nach Anspruch 10, **dadurch gekennzeichnet, dass** die unter den Buchstaben **A, B** und **C** des Verfahrens genannten Schritte wiederholt werden, wobei ber jeder Wiederholung im Vei gleich zui von angegangenen Durchfuhrungen des Verfahrens

Molekul I verandert wird oder ein anderes Molekul als Molekül I bereitgestellt wird, und

Molekul II nicht verandert wird und auch kein anderes Molekül als Molekül II bereitgestellt wird, und umfassendden zusatzlichen Schritt

**D0** Vergleich dei bei den Wiederholungen erhaltenen Gradienten-Norm C3 für die verschiedenen Vorstufen für den Ubeigangszustand und Auswahl dei Vorstufe für den Übergangszustand mit der niedrigsten Gradienten-Norm C3 und Durchführung von Schritt D1 und/oder D2 mit der ausgewahlten Vorstufe für den Ubergangszustand

12. Verfahren nach einem der Anspruche 10 oder 11, **dadurch gekennzeichnet, dass** Molekül I ein Katalysator für die chemische Reaktion ist, insbesondere für eine Polymersynthese, und Molekül II ein Edukt der chemischen Reaktion.

13. Verfahren nach einem der Ansprüche 10 bis 12, **dadurch gekennzeichnet, dass** die Lange des in Schritt **A2** ausgewahlten Abstands zwischen mindestens einem Atom aus Molekul I und/oder mindestens einem Atom aus Molekül II insbesondere höchstens 230 pm betragt

14. Verfahren nach einem der Ansprüche 10 bis 13, **dadurch gekennzeichnet, dass** Molekül I eine Große von 2 bis 1000 Atomen aufweist und Molekül II eine Große von 2 bis 1000 Atomen aufweist, bevorzugt soll die Summe der Atome aus Molekül I und aus Molekül II mehr als 100 Atome betragen.

15. System zur Datenverarbeitung umfassend Mittel zur Ausfuhrung eines Verfahrens umfassend die Schritte

### A Erzeugen einer Startgeometrie

A1 Bereitstellung der dreidimensionalen Darstellung mindestens eines Molekuls im energetischen Grundzustand,
A2 Auswahl von mindestens einer Bindung des mindestens einen Molekuls und Auswahl einer Lange dei Bindung, wobei die ausgewählte Lange nicht der Lange der Bindung im enei getischen Grundzustand des Molekuls entspricht, so dass eine Startgeometrie für die chemische Reaktion erhalten wird,
A3 Dreidimensionale Darstellung der Startgeometrie in kartesischen und/oder internen Koordinaten,

### B Ermittlung einer optimierten Startgeometrie

B1 Festlegung eines Funktionsraumes welcher die mindestens eine Bindung aus Schritt A2 und die durch diese Bindung veibundenen Atome umfasst,

B2 Geometrieoptimierung des in Schritt B1 ausgewählten Funktionsraums mittels einer quantenchemischen Methode und mit der Randbedingung, dass die in Schritt A2 ausgewählte Lange dei mindestens einen Bindung konstant gehalten wird, so dass eine optimierte Startgeometrie erhalten wird,
B3 Ermittlung der Gradient-Norm B3 für die optimierte Startgeometrie, wobei die Gradient-Norm durch die eiste Ableitung einer Funktion E = f(x) der quantenchemischen Methode und erhalten wird, mit E=Gesamtenergie der optimierten Startgeometrie und x=Kernkoordinaten des Molekuls in der optimierten Startgeometrie,
B4 1 wenn die Gradient-Noim B3 $\nabla$ $0 \leq \nabla \leq$ 0,03 $E_h\, a_0^{-1}$ betragt, dann Einordnung dei optimierten Startgeometrie als eine Vorstufe für den Ubergangszustand der chemischen Reaktion und Fortführung des Verfahrens mit Schritt D1, oder
B4 2 wenn die Gradient-Norm B3 $\nabla$ > 0,03 $E_h\, a_0^{-1}$ betragt, dann Ermittlung der Vorstufe für den Ubergangszustand der chemischen Reaktion ausgehend von dei optimierten Startgeometrie durch ein Verfahren umfassend die folgenden Schritte.

### C Ermittlung der Vorstufe für den Übergangszustand der chemischen Reaktion

C1 Variation der optimierten Startgeometrie unter Anwendung eines Monte-Carlo-Algorithmus wobei

C1.1 wenigstens ein Atom aus dem in Schritt B1 ausgewahlten Funktionsraum zufällig ausgewählt wird,
C1 2 ein Vektor für eine Auslenkung des in Schritt C1 gewahlten Atoms zufällig ausgewählt wird, wobei der zufällig ausgewählte Betrag des Vektors mit dei Gradient-Norm B3 gewichtet wird,
C1 3 das in Schritt C1 1 ausgewählte Atom anhand des Vektors aus Schritt C1 2 aus der Position des Atoms in dei optimierten Startgeometrie ausgelenkt wird, so dass eine Vorstufe für einen Übergangszustand der chemischen Reaktion erhalten wird,

C2 Geometrieoptimierung dei Vorstufe für den Ubergangszustand mittels der quantenchemischen Methode und mit dei Randbedingung, dass die mindestens eine Bindung aus Schritt A2 die Lange hat, die sie in der Schritt C3 ermittelt wurde,
C3 Ermittlung der Gradient-Norm C3 für die

Vorstufe für den Übergangszustand aus Schritt C4, wobei die Gradient-Norm durch die eiste Ableitung der Funktion E = f(x) der quantenchemischen Methode und erhalten wird, mit E=Gesamtenergie der Vorstufe für den Übergangszustand und x=Kernkoordinaten des Moleküls in dei Vorstufe für den Übergangszustand,

C4 1 wenn die Gradient-Norm C3 $\nabla$ $0 \leq \nabla \leq$ 0,03 $E_h a_0^{-1}$ betragt, dann Fortführung des Verfahrens mit Schritt D1, oder

C4.2 wenn die Gradient-Norm C3 $\nabla > 0{,}03$ $E_h a_0^{-1}$ betragt, dann Wiederholung dei Schritte C1 bis C3 bis eine Gradient-Norm C3 $\nabla$ $0 \leq \nabla \leq 0{,}03$ $E_h a_0^{-1}$ erhalten wird, wobei

(a) für den Fall, dass die Schritte C1 bis C3 ein Mal durchgeführt wurden, in Schritt C1 die Geometrie der Voi stufe für den Übergangszustand variiert wird, wenn der Wert ihrer Gradient-Norm C3 niedriger ist als dei Wert dei Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vei gleich zu allen bisher erhaltenen Gradient-Normen C3 und B3 den niedrigsten Wert fui die Gradient-Norm C3 oder B3 aufweist,

## D Ermittlung des Übergangszustandes

D1 Variation der Vorstufe aus Schritt C4.1 oder der Vorstufe aus Schritt B4 1, indem folgende Schritte für ein Atom innerhalb des in B1 definierten Funktionsraums durchgeführt werden

D1 1 Auswahl eines Atoms aus dem in Schritt B1 ausgewahlten Funktionsraum,

D1 2 Auswahl eines Vektors für eine Auslenkung des in Schutt D1 1 gewahlten Atoms

D1 3 Auslenkung des in Schritt D1 1 ausgewählten Atoms anhand des Vektors aus Schritt D1 2 aus seiner Position in der Vorstufe, wobei die Position einmal um einen positiven Wert des Betrags des Vektors und einmal um einen negativen Wert des Betrags des Vektors ausgelenkt wird, so dass bei der Durchführung der Schritte D1.1 bis D 1 3 zwei ausgelenkte Vorstufen erhalten werden,

D2 Geometrieoptimierung dei zwei ausgelenkten Vorstufen aus Schritt D1 mittels dei quantenchemischen Methode unter der Randbedingung dass die in D1 erhaltenen Bmdungsabstande konstant gehalten weiden, so dass zwei optimierte Vorstufen (i) und (ii) erhalten werden,

D3 Berechnung dei Energie dei zwei optimierten Vorstufen (i) und (ii) aus Schritt D2 mittels des quantenchemischen Verfahrens,

D4 Vergleich des Wertes der Energie der zwei optimierten Vorstufen mit dem Wert dei Energie C3 oder B3 dei Vorstufe, die in Schritt D1 verwendet wurde,

D4 1 wenn der Weit dei Energie der optimierten Vorstufe (i) und der Wert der Energie dei optimierten Vorstufe (ii) kleiner sind als der Wert dei Energie C3 oder B3 dei Vorstufe, die in Schritt D1 vei wendet wurde, dann Einordnung dei Vorstufe, die in Schritt D1 verwendet wurde, als Übergangszustand,

D4.2 wenn der Wert dei Energie dei optimierten Vorstufe (i) oder der Wert der Energie der optimierten Vorstufe (ii) nicht jeweils kleiner ist als der Weit dei Energie C3 oder B3 der Vorstufe, die in Schritt D1 verwendet wurde, dann Wiederholung des Verfahrens ab Schritt C.

16. Computerprogramm, umfassend Befehle, die bei der Ausführung des Programms durch einen Computer diesen veranlassen, die folgenden Schritte eines Verfahrens durchzufuhren

## Optional A Erzeugen einer Startgeometrie

A1 Bereitstellung dei dreidimensionalen Darstellung mindestens eines Moleküls im energetischen Grundzustand,

A2 Auswahl von mindestens einer Bindung des mindestens einen Moleküls und Auswahl einer Lange der Bindung, wobei die ausgewahlte Lange nicht der Lange der Bindung im energetischen Grundzustand des Moleküls entspricht, so dass eine Startgeometrie für die chemische Reaktion erhalten wird,

A3 Dreidimensionale Darstellung dei Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung des in Schritt B1 ausgewahlten Funktionsraums mittels einer quantenchemischen Methode und mit der Randbedingung, dass die in Schritt A2 ausgewählte Lange dei mindestens einen Bindung konstant gehalten wird, so dass eine optimierte Startgeometrie erhalten wird,

B3 Ermittlung dei Gradient-Norm B3 für die optimierte Startgeometrie, wobei die Gradient-Norm durch die eiste Ableitung einer Funktion $E = f(x)$ dei quantenchemischen Methode und erhalten wird, mit E=Gesamtenergie der optimierten Startgeometrie und x=Kernkoordinaten des Moleküls in dei optimierten Startgeometrie,

B4 1 wenn die Gradient-Norm B3 $\nabla$ $0 \leq \nabla \leq$ 0,03 $E_h$ $a_0^{-1}$ betragt, dann Einordnung der optimierten Startgeometrie als eine Vorstufe für den Übergangszustand der chemischen Reaktion und Fortführung des Verfahrens mit Schritt D1, oder

B4 2 wenn die Gradient-Norm B3 $\nabla$ > 0,03 $E_h$ $a_0^{-1}$ betragt, dann Ermittlung dei Vorstufe für den Ubeigangszustand dei chemischen Reaktion ausgehend von der optimierten Startgeometrie durch ein Verfahren umfassend die folgenden Schritte

**C Ermittlung der Vorstufe für den Übergangszustand der chemischen Reaktion**

C1 Variation der optimierten Startgeometrie unter Anwendung eines Monte-Carlo-Algorithmus wobei

C11 wenigstens ein Atom aus dem in Schritt B1 ausgewahlten Funktionsiaum zufällig ausgewahlt wird,

C12 ein Vektor für eine Auslenkung des in Schritt C1 gewählten Atoms zufällig ausgewählt wird, wobei der zufällig ausgewahlte Betrag des Vektors mit der Gradient-Norm B3 gewichtet wird,

C13 das in Schutt C11 ausgewählte Atom anhand des Vektors aus Schritt C12 aus dei Position des Atoms in der optimierten Startgeometrie ausgelenkt wird, so dass eine Vorstufe für einen Ubergangszustand dei chemischen Reaktion erhalten wild,

C2 Geometrieoptimierung dei Vorstufe für den Ubergangszustand mittels der quantenchemischen Methode und mit der Randbedingung, dass die mindestens eine Bindung aus Schritt A2 die Lange hat, die sie in dei Schritt C3 ermittelt wurde,

C3 Ermittlung der Gradient-Norm C3 für die Vorstufe für den Ubergangszustand aus Schritt C4, wobei die Gradient-Norm durch die erste Ableitung der Funktion $E = f(x)$ dei quantenchemischen Methode und erhalten wird, mit E=Gesamtenergie der Vorstufe für den Ubergangszustand und x=Kernkoordinaten des Molekuls in der Vorstufe für den Ubergangszustand,

C4 1 wenn die Gradient-Norm C3 $\nabla$ $0 \leq \nabla \leq$ 0,03 $E_h$ $a_0^{-1}$ betragt, dann Fortführung des Verfahrens mit Schritt D1, oder

C4.2 wenn die Gradient-Norm C3 $\nabla$ > 0,03 $E_h$ $a_0^{-1}$ betragt, dann Wiederholung der Schritte C1 bis C3 bis eine Gradient-Norm C3 $\nabla$ $0 \leq \nabla \leq$ 0,03 $E_h$ $a_0^{-1}$ erhalten wird, wobei

(a) für den Fall, dass die Schritte C1 bis C3 ein Mal durchgefuhrt wurden, in Schritt C1 die Geometrie dei Vorstufe für den Übergangszustand variiert wird, wenn dei Wert ihrei Gradient-Norm C3 niedriger ist als der Wert der Gradient-Norm B3 der optimierten Startgeometrie, oder

(b) fur den Fall dass die Schritte C1 bis C3 mehr als ein Mal durchgefuhrt wurden, in Schritt C1 die Geometrie dei optimierten Startgeometrie oder derjenigen Vorstufe für den Ubergangszustand aus den vorrangegangen Wiederholungen variiert wird, welche im Vergleich zu allen bisher erhaltenen Gradient-Normen C3 und B3 den niedrigsten Wert fur die Gradient-Norm C3 oder B3 aufweist,

**D Ermittlung des Übergangszustandes**

D1 Variation dei Vorstufe aus Schritt C4.1 oder dei Vorstufe aus Schritt B4 1, indem folgende Schritte für ein Atom innerhalb des in B1 definierten Funktionsraums durchgefuhrt werden

D11 Auswahl eines Atoms aus dem in Schritt B1 ausgewahlten Funktionsraum,

D12 Auswahl eines Vektors für eine Auslenkung des in Schritt D11 gewählten Atoms

D1.3 Auslenkung des in Schritt D11 ausgewahlten Atoms anhand des Vektors aus Schritt D1.2 aus seiner Position in dei Vorstufe, wobei die Position einmal um einen positiven Wert des Betrags des Vektors und einmal um einen negativen Wert des Betrags des Vektors ausgelenkt wird, so dass bei der Durchfuhrung der Schritte D11 bis D13 zwei ausgelenkte Vorstufen erhalten weiden,

D2 Geometrieoptimierung dei zwei ausgelenkten Vorstufen aus Schritt D1 mittels dei quantenchemischen Methode unter dei Randbedingung dass die in D1 erhaltenen Bindungsabstände konstant gehalten werden, so dass zwei optimierte Voi stufen (i) und (ii) erhalten werden,

D3 Berechnung der Energie der zwei optimierten Vorstufen (i) und (ii) aus Schritt D2 mittels des quantenchemischen Veifahiens,

D4 Vergleich des Wertes dei Energie der zwei optimierten Vorstufen mit dem Wert der Energie C3 oder B3 dei Vorstufe, die in Schritt D1 verwendet wurde,

D41 wenn der Wert dei Energie der optimierten Vorstufe (1) und der Weit der Energie der optimierten Vorstufe (ii) kleiner sind als dei Wert dei Energie C3 oder B3 der Vorstufe, die in Schritt D1 verwendet wurde, dann Einordnung dei Vorstufe, die in Schritt D1 verwendet wurde, als Ubergangszustand,

D42 wenn der Wert der Energie der optimierten Vorstufe (i) oder der Wert der Energie der optimierten Vorstufe (ii) nicht jeweils kleiner ist als dei Wert dei Energie C3 oder B3 der Vorstufe, die in Schritt D1 verwendet wurde, dann Wiederholung des Verfahrens ab Schritt C

17. Computerlesbares Speichermedium, umfassend Befehle, die bei dei Ausführung durch einen Computer diesen veranlassen, die folgenden Schritte eines Verfahrens durchzuführen

**Optional A Erzeugen einer Startgeometrie**

A1 Bereitstellung der dreidimensionalen Darstellung mindestens eines Moleküls im energetischen Grundzustand,

A2 Auswahl von mindestens einer Bindung des mindestens einen Molekuls und Auswahl einer Lange der Bindung, wobei die ausgewählte Lange nicht der Lange der Bindung im energetischen Grundzustand des Molekuls entspricht, so dass eine Startgeometrie fur die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funlctionsraumes welcher die mindestens eine Bindung aus Schritt A2 und die durch diese Bindung verbundenen Atome umfasst,

B2 Geometrieoptimierung des in Schritt B1 ausgewahlten Funktionsraums mittels einer quantenchemischen Methode und mit der Randbedingung, dass die in Schritt A2 ausgewahlte Lange dei mindestens einen Bindung konstant gehalten wird, so dass eine optimierte Startgeometrie erhalten wird,

B3 Ermittlung dei Gradient-Norm B3 fur die optimierte Startgeometrie, wobei die Gradient-Norm durch die eiste Ableitung einer Funktion $E = f(x)$ dei quantenchemischen Methode und erhalten wird, mit E=Gesamtenergie der optimierten Startgeometrie und x=Kernkoordinaten des Moleküls in der optimierten Startgeometrie,

B4 1 wenn die Gradient-Norm B3 $\nabla$ $0 \leq \nabla \leq$ 0,03 $E_h\ a_0^{-1}$ betragt, dann Einordnung der optimierten Startgeometrie als eine Vorstufe für den Ubergangszustand der chemischen Reaktion und Fortführung des Verfahrens mit Schritt D1, oder

B42 wenn die Gradient-Norm B3 $\nabla$ > 0,03 $E_h\ a_0^{-1}$ beträgt, dann Ermittlung dei Vorstufe für den Übergangszustand der chemischen Reaktion ausgehend von der optimierten Startgeometrie durch ein Vei fahren umfassend die folgenden Schritte

**C Ermittlung der Vorstufe für den Übergangszustand der chemischen Reaktion**

C1 Variation dei optimierten Startgeometrie unter Anwendung eines Monte-Carlo-Algorithmus wobei

C11 wenigstens ein Atom aus dem in Schutt B1 ausgewahlten Funktionsraum zufällig ausgewählt wird,

C12 ein Vektor fui eine Auslenkung des in Schritt C1 gewählten Atoms zufällig ausgewählt wird, wobei dei zufällig ausgewählte Betrag des Vektors mit der Gradient-Norm B3 gewichtet wird,
C13 das in Schritt C11 ausgewählte Atom anhand des Vektors aus Schritt C1.2 aus dei Position des Atoms in der optimierten Startgeometrie ausgelenkt wird, so dass eine Vorstufe für einen Ubergangszustand der chemischen Reaktion erhalten wird,

C2 Geometrieoptimierung dei Vorstufe für den Ubergangszustand mittels der quantenchemischen Methode und mit der Randbedingung, dass die mindestens eine Bindung aus Schritt A2 die Lange hat, die sie in der Schutt C3 ermittelt wurde,
C3 Ermittlung der Gradient-Norm C3 fur die Vorstufe für den Übergangszustand aus Schritt C4, wobei die Gradient-Norm durch die eiste Ableitung der Funktion E = f(x) der quantenchemischen Methode und erhalten wird, mit E=Gesamtenergie der Vorstufe für den Ubergangszustand und x=Kernkoordinaten des Moleküls in dei Vorstufe für den Übergangszustand,
C4 1 wenn die Gradient-Norm C3 $\nabla$ $0 \leq \nabla \leq$ 0,03 $E_h$ $a_0^{-1}$ betragt, dann Fortführung des Verfahrens mit Schritt D1, oder
C42 wenn die Gradient-Noim C3 $\nabla$ > 0,03 $E_h$ $a_0^{-1}$ betragt, dann Wiederholung der Schritte C1 bis C3 bis eine Gradient-Norm C3 $\nabla$ $0 \leq \nabla \leq$ 0,03 $E_h$ $a_0^{-1}$ erhalten wird, wobei

(a) für den Fall, dass die Schritte C1 bis C3 ein Mal durchgeführt wurden, in Schritt C1 die Geometrie der Voi stufe für den Ubergangszustand variiert wild, wenn der Wert ihrer Gradient-Norm C3 niedriger ist als der Wert der Gradient-Norm B3 dei optimierten Startgeometrie, oder
(b) fur den Fall dass die Schritte C1 bis C3 mehr als ein Mal durchgefuhrt wurden, in Schritt C1 die Geometrie dei optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert fur die Gradient-Norm C3 oder B3 aufweist,

**D Ermittlung des Übergangszustandes**

D1 Variation der Vorstufe aus Schritt C4 1 oder dei Vorstufe aus Schritt B41, indem folgende Schritte für ein Atom innerhalb des in B1 definierten Funktionsraums durchgeführt werden

D11 Auswahl eines Atoms aus dem in Schritt B1 ausgewahlten Funktionsraum,
D12 Auswahl eines Vektors für eine Auslenkung des in Schritt D11 gewählten Atoms
D13 Auslenkung des in Schritt D11 ausgewahlten Atoms anhand des Vektors aus Schritt D12 aus seiner Position in der Vorstufe, wobei die Position einmal um einen positiven Wert des Betrags des Vektors und einmal um einen negativen Wert des Betrags des Vektors ausgelenkt wird, so dass bei der Durchführung dei Schritte D11 bis D.13 zwei ausgelenkte Vorstufen erhalten weiden,

D2 Geometrieoptimierung dei zwei ausgelenkten Vorstufen aus Schritt D1 mittels der quantenchemischen Methode unter dei Randbedingung dass die in D1 erhaltenen Bindungsabstände konstant gehalten werden, so dass zwei optimierte Vorstufen (i) und (ii) erhalten werden,
D3 Berechnung der Energie dei zwei optimierten Vorstufen (i) und (ii) aus Schritt D2 mittels des quantenchemischen Verfahrens,
D4 Vergleich des Wertes der Energie der zwei optimierten Vorstufen mit dem Wert dei Energie C3 oder B3 der Vorstufe, die in Schutt D1 verwendet wurde,

D4.1 wenn der Wert dei Energie dei optimierten Vorstufe (i) und der Wert der Energie der optimierten Vorstufe (ii) kleiner sind als der Wert der Energie C3 oder B3 der Vorstufe, die in Schritt D1 verwendet wurde, dann Einordnung der Vorstufe, die in Schritt D1 verwendet wurde, als Ubergangszustand,
D42 wenn der Wert dei Energie dei optimierten Vorstufe (i) oder der Wert der Energie der optimierten Vorstufe (ii) nicht jeweils kleiner ist als dei Wert der Energie C3 oder B3 der Vorstufe, die in Schritt D1 verwendet wurde, dann Wiederholung des Verfahrens ab Schutt C

18. Verwendung eines Computerprogramms nach Anspruch 16 oder eines computerlesbaren Speichermediums nach Anspruch 17 zur Bewertung von Übergangszustanden einer chemischen Reaktion, insbesondere einer Polymersynthese

Europäisches
Patentamt

European
Patent Office

Office européen
des brevets

# EUROPÄISCHER RECHERCHENBERICHT

Nummer der Anmeldung

EP 18 20 1344

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | LIN X-X. ET AL: "A flexible transition state searching method for atmospheric reaction systems", CHEMICAL PHYSICS, NORTH-HOLLAND, NL, Bd. 450, 11. Februar 2015 (2015-02-11), Seiten 21-31, XP029204426, ISSN: 0301-0104, DOI: 10.1016/J.CHEMPHYS.2015.02.002 * das ganze Dokument * ----- | 1-18 | INV. G16C20/10 ADD. G16C10/00 |
| A | SWIHART M. T. ET AL: "Assembling gas-phase reaction mechanisms for high temperature inorganic systems based on quantum chemistry calculations and reaction rate theories", JOURNAL OF PHYSICS AND CHEMISTRY OF SOLIDS, PERGAMON PRESS, LONDON, GB, Bd. 66, Nr. 2-4, 1. Februar 2005 (2005-02-01), Seiten 364-371, XP027709615, ISSN: 0022-3697 [gefunden am 2005-02-01] * Seite 365, linke Spalte, Zeile 36 - Seite 366, linke Spalte, Zeile 8 * ----- | 1-18 | |
| A | HAFNER J.: "Adsorption and reaction of organic molecules on solid surfaces - ab-initio density functional investigations", MONATSHEFTE FÜR CHEMIE - CHEMICAL MONTHLY ; AN INTERNATIONAL JOURNAL OF CHEMISTRY, SPRINGER-VERLAG, AU, Bd. 139, Nr. 4, 14. März 2008 (2008-03-14), Seiten 373-387, XP019592602, ISSN: 1434-4475 * Seite 375, rechte Spalte, Zeilen 10-19 * * Abbildungen 1,2,7,8 * ----- -/-- | 1-18 | RECHERCHIERTE SACHGEBIETE (IPC) G16C |

Der vorliegende Recherchenbericht wurde für alle Patentansprüche erstellt

| Recherchenort | Abschlußdatum der Recherche | Prüfer |
|---|---|---|
| Den Haag | 8. Juli 2019 | Godzina, Przemyslaw |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.............................................................
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

**Nummer der Anmeldung**

EP 18 20 1344

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 2 352 107 A1 (UNIV YAMAGUCHI [JP]) 3. August 2011 (2011-08-03) * das ganze Dokument * ----- | 1-18 | |
| A | JP 2008 250392 A (UNIV YAMAGUCHI) 16. Oktober 2008 (2008-10-16) * das ganze Dokument * ----- | 1-18 | |
| A | CN 104 765 918 A (UNIV EAST CHINA SCIENCE & TECH) 8. Juli 2015 (2015-07-08) * das ganze Dokument * ----- | 1-18 | |
| A,D | MARTÍNEZ-NÚÑEZ E.: "An automated transition state search using classical trajectories initialized at multiple minima", PHYSICAL CHEMISTRY CHEMICAL PHYSICS, Bd. 17, Nr. 22, 6. Mai 2015 (2015-05-06), Seiten 14912-14921, XP055601422, ISSN: 1463-9076, DOI: 10.1039/C5CP02175H * das ganze Dokument * ----- | 1-18 | |
| A,D | RODRÍGUEZ A. ET AL: "tsscds2018: A code for automated discovery of chemical reaction mechanisms and solving the kinetics", JOURNAL OF COMPUTATIONAL CHEMISTRY., Bd. 39, Nr. 23, 5. September 2018 (2018-09-05), Seiten 1922-1930, XP055601919, GB ISSN: 0192-8651, DOI: 10.1002/jcc.25370 * das ganze Dokument * ----- | 1-18 | RECHERCHIERTE SACHGEBIETE (IPC) |

-/--

Der vorliegende Recherchenbericht wurde für alle Patentansprüche erstellt

| Recherchenort | Abschlußdatum der Recherche | Prüfer |
|---|---|---|
| Den Haag | 8. Juli 2019 | Godzina, Przemyslaw |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

Seite 2 von 6

Europäisches
Patentamt

European
Patent Office

Office européen
des brevets

## EUROPÄISCHER RECHERCHENBERICHT

Nummer der Anmeldung

EP 18 20 1344

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | JACOBSON L. D. ET AL: "Automated Transition State Search and Its Application to Diverse Types of Organic Reactions", JOURNAL OF CHEMICAL THEORY AND COMPUTATION: JCTC, Bd. 13, Nr. 11, 17. Oktober 2017 (2017-10-17), Seiten 5780-5797, XP055601412, US ISSN: 1549-9618, DOI: 10.1021/acs.jctc.7b00764 * das ganze Dokument * ----- | 1-18 | |
| A | SALCICCIOLI M. ET AL: "A review of multiscale modeling of metal-catalyzed reactions: Mechanism development for complexity and emergent behavior", CHEMICAL ENGINEERING SCIENCE, OXFORD, GB, Bd. 66, Nr. 19, 30. Mai 2011 (2011-05-30), Seiten 4319-4355, XP028264661, ISSN: 0009-2509, DOI: 10.1016/J.CES.2011.05.050 [gefunden am 2011-06-13] * Zusammenfassung * * Abbildungen 11,26,27 * ----- | 1-18 | RECHERCHIERTE SACHGEBIETE (IPC) |
| | -/-- | | |

Der vorliegende Recherchenbericht wurde für alle Patentansprüche erstellt

| Recherchenort | Abschlußdatum der Recherche | Prüfer |
|---|---|---|
| Den Haag | 8. Juli 2019 | Godzina, Przemyslaw |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
......................................................................
& : Mitglied der gleichen Patentfamilie, übereinstimmendes
Dokument

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 18 20 1344

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | KEIL F. J.: "Complexities in modeling of heterogeneous catalytic reactions", COMPUTERS & MATHEMATICS WITH APPLICATIONS, ELSEVIER, AMSTERDAM, NL, Bd. 65, Nr. 10, 3. Januar 2013 (2013-01-03), Seiten 1674-1697, XP028556589, ISSN: 0898-1221, DOI: 10.1016/J.CAMWA.2012.11.023 * Zusammenfassung * * Seite 1688, Zeilen 13-16 * * Seite 1692, Zeilen 15-38 * * Abbildung 19 * | 1-18 | |
| A | DIEDRICH M. K. ET AL: "Experimental Determination of the Activation Parameters and Stereoselectivities of the Intramolecular Diels-Alder Reactions of 1,3,8-Nonatriene, 1,3,9-Decatriene, and 1,3,10-Undecatriene and Transition State Modeling with the Monte Carlo-Jumping Between Wells/Molecular Dynamics Method", JOURNAL OF THE AMERICAN CHEMICAL SOCIETY, Bd. 119, Nr. 43, 1. Oktober 1997 (1997-10-01), Seiten 10255-10259, XP055593729, ISSN: 0002-7863, DOI: 10.1021/ja9643331 * Zusammenfassung * * Seite 10256, rechte Spalte, Zeile 48 - Seite 10259, linke Spalte, Zeile 7 * | 1-18 | RECHERCHIERTE SACHGEBIETE (IPC) |
| A | US 2003/236655 A1 (GOVIND NIRANJAN [US] ET AL) 25. Dezember 2003 (2003-12-25) * das ganze Dokument * | 1-18 | |
| A | JP H11 25063 A (HITACHI LTD) 29. Januar 1999 (1999-01-29) * das ganze Dokument * | 1-18 | |

-/--

Der vorliegende Recherchenbericht wurde für alle Patentansprüche erstellt

| Recherchenort | Abschlußdatum der Recherche | Prüfer |
|---|---|---|
| Den Haag | 8. Juli 2019 | Godzina, Przemyslaw |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes
   Dokument

EPO FORM 1503 03.82 (P04C03)

Seite 4 von 6

Europäisches
Patentamt

European
Patent Office

Office européen
des brevets

**EUROPÄISCHER RECHERCHENBERICHT**

Nummer der Anmeldung

EP 18 20 1344

| | **EINSCHLÄGIGE DOKUMENTE** | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| A | WO 2018/102565 A1 (SCHROEDINGER INC [US]) 7. Juni 2018 (2018-06-07) * das ganze Dokument * ----- | 1-18 | |
| A | MERCERO J. M. ET AL: "Theoretical methods that help understanding the structure and reactivity of gas phase ions", INTERNATIONAL JOURNAL OF MASS SPECTROMETRY, ELSEVIER SCIENCE PUBLISHERS, AMSTERDAM, NL, Bd. 240, Nr. 1, 1. Januar 2005 (2005-01-01), Seiten 37-99, XP027705154, ISSN: 1387-3806 [gefunden am 2005-01-01] * Seite 71, rechte Spalte, Zeilen 10-35 * ----- | 1-18 | |
| A | KRATZER P.: "Monte Carlo and kinetic Monte Carlo methods", ARXIV.ORG, CORNELL UNIVERSITY LIBRARY, 201 OLIN LIBRARY CORNELL UNIVERSITY ITHACA, NY 14853, 16. April 2009 (2009-04-16), XP080320087, * das ganze Dokument * ----- | 1-18 | RECHERCHIERTE SACHGEBIETE (IPC) |
| A | GREBNER C. ET AL: "PathOpt-A global transition state search approach: Outline of algorithm", JOURNAL OF COMPUTATIONAL CHEMISTRY., Bd. 34, Nr. 21, 4. Mai 2013 (2013-05-04), Seiten 1810-1818, XP055601427, GB ISSN: 0192-8651, DOI: 10.1002/jcc.23307 * das ganze Dokument * ----- | 1-18 | |

-/--

Der vorliegende Recherchenbericht wurde für alle Patentansprüche erstellt

| Recherchenort | Abschlußdatum der Recherche | Prüfer |
|---|---|---|
| Den Haag | 8. Juli 2019 | Godzina, Przemyslaw |

EPO FORM 1503 03.82 (P04C03)

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.........................................................................
& : Mitglied der gleichen Patentfamilie, übereinstimmendes Dokument

Seite 5 von 6

| | Europäisches Patentamt European Patent Office Office européen des brevets | **EUROPÄISCHER RECHERCHENBERICHT** | **Nummer der Anmeldung** EP 18 20 1344 |
|---|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | LIN Y. ET AL: "Reliable Modeling and Optimization for Chemical Engineering Applications: Interval Analysis Approach", RELIABLE COMPUTING ; AN INTERNATIONAL JOURNAL DEVOTED TO RELIABLE MATHEMATICAL COMPUTATIONS BASED ON FINITE REPRESENTATIONS AND GUARANTEED ACCURACY, KLUWER ACADEMIC PUBLISHERS, DO, Bd. 12, Nr. 6, 25. Oktober 2006 (2006-10-25), Seiten 427-450, XP019453859, ISSN: 1573-1340, DOI: 10.1007/S11155-006-9013-6 * Seite 442, Absatz 5. - Seite 447, Absatz 6. * ----- | 1-18 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Den Haag | 8. Juli 2019 | Godzina, Przemyslaw |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...................................................................
& : Mitglied der gleichen Patentfamilie, übereinstimmendes
    Dokument

EPO FORM 1503 03.82 (P04C03)

Seite 6 von 6

**ANHANG ZUM EUROPÄISCHEN RECHERCHENBERICHT
ÜBER DIE EUROPÄISCHE PATENTANMELDUNG NR.**

EP 18 20 1344

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-07-2019

| Im Recherchenbericht angeführtes Patentdokument | Datum der Veröffentlichung | Mitglied(er) der Patentfamilie | Datum der Veröffentlichung |
|---|---|---|---|
| EP 2352107 A1 | 03-08-2011 | EP 2352107 A1<br>JP 5164111 B2<br>JP 2010097371 A<br>US 2011191390 A1<br>WO 2010044191 A1 | 03-08-2011<br>13-03-2013<br>30-04-2010<br>04-08-2011<br>22-04-2010 |
| JP 2008250392 A | 16-10-2008 | JP 4324680 B2<br>JP 2008250392 A | 02-09-2009<br>16-10-2008 |
| CN 104765918 A | 08-07-2015 | KEINE | |
| US 2003236655 A1 | 25-12-2003 | CA 2431474 A1<br>US 2003236655 A1 | 24-12-2003<br>25-12-2003 |
| JP H1125063 A | 29-01-1999 | KEINE | |
| WO 2018102565 A1 | 07-06-2018 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **LIN et al.** A flexible transition state searching method for atmospheric reaction systems. *Chemical Physics,* 2015, vol. 450-451, 21-31
- **E MARTINEZ-NÚÑEZ et al.** An automated transition state search using classical trajectories initialized at multiple minima. *Phys Chem Chem Phys,* 14. Juni 2015, vol. 17 (22), 14912-21
- tsscds2018: A code foi automated discovery of chemical reaction mechanisms and solving the kinetics. *J. Comp Chem,* 24. September 2018, vol. 39 (23), 1922-1930

- **JACOBSON et al.** Automated Transition State Search and Its Application to Diverse Types of Organic Reactions. *J. Chem Theory Comput.,* vol. 13 (11), 5780-5797
- **HU et al.** A gradient-directed Monte Carlo method for global optimization in a discrete space Application to protein sequence design and folding. *J Chem Phys,* 21. Oktober 2009, vol. 131 (15), 154117